# EUROPÄISCHE PATENTSCHRIFT

(11) **EP 2 922 460 B1**
(45) Veröffentlichungstag und Bekanntmachung des Hinweises auf die Patenterteilung: **14.09.2016**
(21) Anmeldenummer: 13799503.1
(22) Anmeldetag: 20.11.2013
(51) Int. Cl.: A61B 3/09, A61B 3/08, A61B 3/032

(54) **VORRICHTUNG SOWIE VERFAHREN ZUR ÜBERPRÜFUNG DER MENSCHLICHEN SEHFÄHIGKEIT**
DEVICE AND METHOD FOR CHECKING HUMAN VISION
DISPOSITIF AINSI QUE PROCÉDÉ DE CONTRÔLE DE L'ACUITÉ VISUELLE HUMAINE

(30) Priorität: 21.11.2012 DE 102012022662
(43) Veröffentlichungstag der Anmeldung: 30.09.2015
(73) Patentinhaber: Voigtmann GmbH, 90443 Nürnberg (DE); Talkingeyes&more GmbH, 91052 Erlangen (DE); Friedrich-Alexander-Universität Erlangen-Nürnberg, 91054 Erlangen (DE); Universitätsklinikum Erlangen, 91054 Erlangen (DE)
(72) Erfinder: VOIGTMANN, Peter, 90607 Rückersdorf (DE); HÖHER, Bernhard, 91054 Erlangen (DE); SCHMAUSS, Bernhard, 92637 Theisseil (DE); MICHELSON, Georg, 91083 Baiersdorf (DE)
(74) Vertreter: Stippl, Hubert
(86) Internationale Anmeldenummer: PCT/EP2013/074290
(87) Internationale Veröffentlichungsnummer: WO 2014/079885

(56) Entgegenhaltungen:
- EP-A1- 2 329 761
- US-A1- 2005 174 536
- WOLFGANG JASCHINSKI: "Asthenopische Beschwerden und die Konvergenz der Augen am Bildschirmarbeitsplatz: Neue Messverfahren fuer Praxis und Forschung", KLINISCHE MONATSBLAETTER FUER AUGENHEILKUNDE, FERDINAND ENKE VERLAG, STUTTGART, DE, Bd. 220, Nr. 8, 1. Januar 2003 (2003-01-01), Seiten 551-558, XP009176364, ISSN: 0023-2165

## Beschreibung

Die Erfindung betrifft eine Vorrichtung zur Überprüfung der menschlichen Sehfähigkeit gemäß Anspruch 1 und ein entsprechendes Verfahren gemäß Anspruch 12.

### Technologischer Hintergrund

Das binokulare menschliche Sehvermögen wird maßgeblich bestimmt durch die Augenmotilität, die Fixationsfähigkeit, die Konvergenz- und Fusionsfähigkeit, die Akkommodationsfähigkeit und die Fähigkeit des stereoskopischen Sehens. Die zugrundeliegenden Funktionen sind durch physiologische und neurologische Vorgänge miteinander verbunden und teilweise voneinander abhängig.

Die Fixationsfähigkeit bestimmt, inwieweit jedes Auge auf einen Fixationspunkt, z.B. einen Gegenstand, ausgerichtet werden und einem bewegten Objekt folgen kann. Durch einen neurologischen Regelkreis wird dabei das Auge über die Augenmuskulatur so gedreht, dass die Abbildung des Fixationspunktes auf der Retina (Netzhaut) stets auf der Fovea Centralis (=Sehgrube) zu liegen kommt.

Die Konvergenzfähigkeit ist abhängig davon, inwieweit beide Augen beim binokularen Sehen auf den gleichen Fixationspunkt ausgerichtet werden können. Im Normalfall verlaufen die Blickrichtungen beider Augen bei der Betrachtung eines unendlich weit entfernten Objekts parallel. Bei geringerem Objektabstand ergibt sich zwischen beiden Blickrichtungen ein in Abhängigkeit des Objektabstandes variierender Konvergenzwinkel. Durch neurologische Regelkreise wird dabei die Verformung der Augenlinse durch den Ziliarmuskel so geregelt, dass der Fixationspunkt scharf auf die Fovea Centralis abgebildet wird.

Bei der Fusion handelt es sich um einen neurologischen Vorgang, bei dem die Bilder beider Augen im Gehirn zu einem Gesamtbild vereinigt werden. Es entsteht ein "3D-Eindruck". Dieser Prozess spielt sich im Gehirn ab und kann von außen nicht beobachtet werden. Die Fähigkeit des räumlichen oder stereoskopischen Sehens (Stereopsis) beruht auf der Fähigkeit des Gehirns aus den Unterschieden zwischen den beiden Bildern auf Basis des eingestellten Konvergenzwinkels eine Tiefeninformation zu ermitteln.

Die Akkommodationsfähigkeit bestimmt, in welchem Maß die Brechkraft der Augenlinse mittels des Ziliarmuskels verändert und dem jeweils betrachteten Objektabstand angepasst werden kann, sodass Objekte aus unterschiedlicher Entfernung auf die Netzhaut scharf abgebildet werden können. Im entspannten Zustand des Auges werden beim normalsichtigen Auge weit entfernte Objekte scharf auf die Netzhaut abgebildet.

Die akkommodative Konvergenz bezeichnet die Ausrichtung beider Augen auf denselben Fixationspunkt. Dabei sind die Augen bei nahen Objekten in Richtung Nase gedreht, sodass sich die beiden Gesichtslinien annähern (konvergieren) und sich schließlich im Objekt schneiden. Der Konvergenzwinkel unter dem sich die Gesichtslinien schneiden, ist umso größer, je kleiner der Abstand zwischen Beobachter und Objekt ist.

Zwei neurophysiologische Prozesse bestimmen die Akkommodationsfähigkeit der Augen. Der eine beruht auf dem Autofokusprinzip, das auch in Kameras zum Einsatz kommt. Die Einstellung des Auges wird leicht variiert und es wird geprüft, ob sich die Bildschärfe verbessert oder verschlechtert hat. Abhängig vom Ergebnis wird die Einstellung wieder rückgängig gemacht oder mit der Verschiebung fortgefahren bis ein Optimum gefunden ist. Dieser Prozess ermöglicht das Einstellen des Auges beim monokularen Sehen auf Objekte in unterschiedlicher Entfernung. Beim binokularen Sehen wird ein zweiter, als Naheinstellungstrias bezeichneter neurophysiologischer Prozess wirksam. Akkommodative Konvergenz und Akkommodation stehen zueinander in einem festen Verhältnis, das durch den sogenannten AC/A-Quotienten beschrieben wird. Diesen konstanten optimalen Wert "versucht" die Naheinstellungstrias stets einzustellen. Ist der Konvergenzwinkel bekannt, lässt sich daraus die Entfernung des angeschauten Gegenstandes ermitteln und aus der Entfernung auch die erforderliche Akkommodation. Dieser Zusammenhang wird im Gehirn durch die neurophysiologische Kopplung von Konvergenz und Akkommodation genutzt.

In ähnlicher Weise charakterisiert der CA/C-Quotient das Verhältnis von Konvergenzakkommodation (CA) zur Konvergenz (C).

### Nächstliegender Stand der Technik

Im Patent WO 2011/107244 A1 ist ein Gerät zur subjektiven Refraktionsbestimmung beschrieben, bei dem gleichzeitig mehrere Objekte mit unterschiedlichen Fokuslagen erzeugt und dem Probanden zum Vergleich angezeigt werden. In der binokularen Variante wird jedoch die für das natürliche Sehen erforderliche Konvergenz von Objekten in unterschiedlichen Fokuslagen nicht berücksichtigt. Die beiden Vergleichszeichen werden damit nicht realistisch dargestellt. Eine Störung der Naheinstellungstrias und eine Verfälschung der Bestimmung der subjektiven Refraktion sind daher möglich.

Eine Vorrichtung und ein Verfahren zur Ermittlung der subjektiven Refraktion beim binokularen Sehen sind auch im Patent US 2008/0246921 A1 beschrieben. Mit dieser Vorrichtung ist zwar die Stimulation der Akkommodation möglich, jedoch werden Sehtesttafeln eingesetzt, mit denen kein Stimulus für die Konvergenz erzeugt werden kann. Es ist auch keine Blickrichtungsmesseinrichtung vorhanden, mit der die Augenstellung gemessen werden könnte. Ein korrekter Stimulus für die Naheinstellungstrias kann somit nicht garantiert werden. Gerade für eine subjektive binokulare Refraktionsmessung kann jedoch die korrekte Stimulation der Naheinstellungstrias als binokularer, neurologischer Regelkreis von entscheidender Bedeutung sein.

In der Patentschrift DE 195 01 415 C2 wird ein Freisichtgerät beschrieben, bei dem in die reale Umgebung virtuelle Testobjekte eingeblendet werden können. Es handelt sich um ein binokulares Gerät, welches Akkommodation und Konvergenz korrekt simuliert. Allerdings sind Akkommodation und Konvergenz fest gekoppelt, sodass der AC/A-Quotient nicht variierbar ist. Das ist problematisch, weil aufgrund von unterschiedlichen Augenabständen und anderer Faktoren der individuelle AC/A-Wert von Mensch zu Mensch schwankt. Bei Menschen mit besonders großem oder besonders kleinem Augenabstand ist somit mit einer Störung der Naheinstellungstrias zu rechen. Unnatürliche AC/A-Quotienten, welche etwa bei virtuellen Umgebungen, z.B. beim 3D-Kino oder 3D-Fernsehen auftreten, können ebenfalls nicht simuliert werden. Außerdem können die Einstellungen nicht getrennt für beide Augen vorgenommen werden. Die Akkommodation kann mit dem Gerät nicht gemessen werden, die Konvergenz kann nur indirekt und subjektiv bestimmt werden.

Das Patent US 5825456 A beschreibt ein Gerät zur Erzeugung stereoskopischer Bilder, bei denen Konvergenz und Größe des Objekts variiert und mit Referenzobjekten verglichen werden. Es soll damit untersucht werden, wie ein Proband relative und absolute Distanzen subjektiv wahrnimmt. Die Vorrichtung ist Brillenförmig und erzeugt ein variables stereoskopisches Sehzeichen mittels Displays. Gleichzeitig werden mit Eye-trackern ("gazing point dectecting") die Fixationspunkte beider Augen bestimmt. Es findet jedoch keine Variation der Fokuslage statt. Der fehlende Akkommodations-Stimulus führt somit zu einem unnatürlichen AC/A-Wert. Somit wird ein unnatürlicher Sehzustand erzeugt, der die Naheinstellungstrias stört. Damit ist es auch wahrscheinlich, dass das im Patent untersuchte subjektive Entfernungsempfinden dadurch verfälscht wird.

In einer Veröffentlichung zu einer klinischen Studie wird ein "Shutter-Brillen-Haploskop mit Eye-Tracker-Regelung zum Zweck der Fusionstherapie" beschrieben, vgl. Klinische Monatsblätter Augenheilkunde 2003; 220: 629-633. Mit einer Shutter-Brille und einem Computermonitor wird ein stereoskopisches Bild erzeugt. Mittels Eye-trackern wird die Fixation und Konvergenz der Augen gemessen. Für die Konvergenz liegen ein Stimulus und eine Messung vor. Über einen Computer findet eine Rückkopplung statt. Die Akkommodation wird nicht berücksichtigt. Deshalb korrespondieren Akkommodation und Konvergenz nicht, eine Störung der Naheinstellungstrias ist möglich, was zu einer Verfälschung der Resultate der Studie geführt haben kann.

Die oben beschriebenen Verfahren haben alle das Problem, dass bei der Simulation einer virtuellen dreidimensionalen Umgebung oder eines Sehzeichens ein unnatürlicher AC/A-Quotient hervorgerufen wird. Der AC/A-Quotient, d.h. die Kopplung von Augenkonvergenz und Akkommodation wird entweder überhaupt nicht berücksichtigt oder ist nur für Menschen mit einem bestimmten Augenabstand korrekt eingestellt. Dadurch kann es zu einer Störung der Naheinstellungstrias und somit einer Verfälschung von Messergebnissen kommen. Außerdem wird bei den beschriebenen Verfahren/Anordnungen entweder die Konvergenz oder die Akkommodation untersucht.

Die DE 197 04 197 A1 beschreibt eine mono- oder binokulare Anordnung zur subjektiven Refraktionsbestimmung sowie anderer Sehfunktionen unter Verwendung einer mit Laserscannern versehenen Probierbrille. Die Anordnung dient zur subjektiven Refraktionsbestimmung. Bei der binokularen Variante der Anordnung ist für jedes Auge ein Strahlengang zur Erzeugung eines Bilds auf der Netzhaut des Probanden vorgesehen, sodass die Bilder gleichzeitig oder abwechselnd dargeboten werden können. Bei dem binokularen Seheindruck werden zwei Bilder zu einem fusioniert.

Die JP 1 164 351 A beschreibt einen ophthalmoskopischen Apparat, bei dem jedem Auge ein optisches System zugeordnet ist, welches jeweils zur Untersuchung mittels verschiebbarer Träger dem Augenabstand sowie den Fixationspunkten angepasst werden kann.

Die US 2005/0174536 A1 beschreibt eine dem Gegenstand des Schutzbegehrens entsprechende Überprüfung der menschlichen Sehfähigheit.

Viele orthoptische Untersuchungen erfolgen heute manuell durch subjektive Methoden. Durch die Subjektivität ist eine Vergleichbarkeit von Ergebnissen unterschiedlicher Untersuchungseinrichtungen nicht uneingeschränkt möglich. Eine unmittelbare Anbindung an EDV-Geräte und die Nutzung der damit verbundenen Möglichkeiten einer Fernbefundung (z.B. Telemedizin) fehlen. Mit den oben genannten Geräten sind nur "Schnappschuss"-Messungen möglich, zeitlich kontinuierliche Messungen sind nicht möglich.

Subjektive Verfahren ermöglichen nur eine begrenzte Anzahl an Messungen in relativ großen zeitlichen Abständen. Eine zeitlich hochauflösende Analyse des Verlaufs z.B. der Akkommodation im Vergleich zum Stimulus ist nicht möglich. Auftretende Einschwingvorgänge der Konvergenz oder der Akkommodation sind mit den genannten Geräten nicht messbar.

Die genannten Geräte erlauben nicht die Durchführung von dynamischen Aufdeck- und Abdecktests zur Feststellung von Tropien und Phorien.

### Aufgabe der vorliegenden Erfindung

Die Aufgabe der Erfindung besteht darin, eine verbesserte Vorrichtung zur Überprüfung der menschlichen Sehfähigkeit sowie ein entsprechendes Verfahren zur Verfügung zu stellen.

### Lösung der Aufgabe

Die Aufgabe wird durch eine Vorrichtung mit den Merkmalen des Anspruchs 1 und ein Verfahren mit den Merkmalen des Anspruchs 11 gelöst.

Die vorliegende Erfindung ermöglicht es, eine fortlaufende Reihe von gleichzeitigen oder quasi-gleichzeitigen Messdaten der Akkommodation sowie Blickrichtung eines Auges zu erzeugen und einer Auswertung zuzuführen. Vor allem können auch die Reaktionszeiten des menschlichen Sehapparates auf Stimuli unterschiedlichster Art geprüft werden. Es besteht die Möglichkeit, zeitlich kontinuierliche Messungen, insbesondere auch über große Zeitabstände durchzuführen. Auftretende Einschwingvorgänge der Akkommodation sowie der Fixationsfähigkeit werden messbar. Die Erfindung ermöglicht zudem zeitlich hochauflösende Analysen des Verlaufs bestimmter Größen, z. B. der Akkommodation im Vergleich zum Stimulus. Dynamische Auf- und Abdecktests zur Diagnose von Tropien und/oder Phorien werden durchführbar. Die Erfindung ermöglicht es, neurophysiologische Prozesse und Regelkreise besser zu erforschen als dies bisher möglich war.

Die in Anspruch 2 beanspruchte Vorrichtung ermöglicht es, die Augenmotilität, die Fixationsfähigkeit, die Akkommodation sowie die akkommodative Konvergenz in voneinander unabhängiger Weise zu ermitteln und die Stereopsis, die Naheinstellungstrias und den AC/A-Quotienten und/oder den CA/C-Quotienten in voneinander unabhängiger Weise zu untersuchen und insbesondere auszugeben.

Es wird ein Untersuchungsgerät zur Verfügung gestellt, mit dem gleichzeitig, unabhängig voneinander und mit der Möglichkeit einer hohen zeitlichen Auflösung, die binokulare Beobachtung, Messung und Dokumentation der Augenmotilität, der Fixationsfähigkeit, der Akkommodation, der akkommodativen Konvergenz und die Untersuchung von Stereopsis und Naheinstellungstrias sowie die Bestimmung des AC/A-Quotienten in objektiver Weise durchgeführt werden kann. Eine zeitgleiche Untersuchung beider Größen in Verbindung mit der Möglichkeit zur Erzeugung entsprechender Stimuli ermöglicht es, umfassende Informationen über den strabologischen Zustand des Probanden zu gewinnen. Auftretende Einschwingvorgänge der Konvergenz werden messbar.

Zweckmäßigerweise erfolgt mittels eines Steuerungs- und Auswertemoduls die Erzeugung von Testbildern auf Bilderzeugungsmodulen, die Einstellung der optischen Komponenten von Abbildungsmodulen, die Steuerung der Messwerterfassung mittels einer Akkommodationsmesseinrichtung, die Steuerung der Messwerterfassung mittels einer Blickrichtungsmesseinrichtung und/oder die Auswertung, Visualisierung und/oder Speicherung der Messwerte.

Die Erfindung bietet folgende gerätetechnische und verfahrenstechnische Vorteile gegenüber den bisher bekannten ophthalmologischen Untersuchungsgeräten und-verfahren, jeweils für sich gesehen aber auch in Kombination bzw. Teilkombination:

Die Erfindung ermöglicht insbesondere die Erzeugung einer variablen virtuellen dreidimensionalen Umgebung, mit der unterschiedliche Stimuli für die Konvergenz und/oder die Akkommodation des menschlichen Sehapparates generiert werden können. Die Stimuli für die Konvergenz und die Akkommodation des menschlichen Sehapparates sind unabhängig voneinander einstellbar, so dass die Stimulation mit unterschiedlichen AC/A-Quotienten und/oder CA/C-Quotienten erfolgen kann.

Beispielsweise kann durch die Wahl eines an den Probanden angepassten AC/A-Quotienten ein natürlicher Seheindruck simuliert werden. Alternativ kann bei Bedarf gezielt ein davon abweichender AC/A-Quotient eingestellt werden, um gezielt eine Reaktion auf einen unnatürlichen Stimulus zu testen.

Die Stimuli werden insbesondere mittels einer computerbasierten Steuerung generiert bei gleichzeitiger bzw. quasigleichzeitiger Messung von Konvergenz und Akkommodation sowie der computergestützten Erfassung, Auswertung und Visualisierung der Messwerte.

Zweckmäßigerweise können die erzeugten Stimuli und die Reaktionen der Augen auf die Stimuli in grafischen Darstellungen gegenüberstellt werden.

Zur Stimulation des Sehapparates können insbesondere beliebige Testfiguren für die Fixation der Augen, die Konvergenz und Akkommodation sowie für Stereopsis und Naheinstellungstrias automatisch, flexibel und in kürzesten Zeitabständen computergesteuert zur Anwendung gebracht werden.

Vorzugsweise kann aus einer Vielzahl von Messgrößen und/oder berechneten Werten eine Kennzahl berechnet werden. Diese kann beispielsweise in eine geeignete Bewertungsskala eingeordnet werden.

Das Sehtestgerät ermöglicht insbesondere binokulare Messungen der Akkommodation, die Erfassung der Fixationspunkte beider Augen, die Bestimmung der Konvergenz, des AC/A-Quotienten und/oder des CA/C-Quotienten und/oder die visualisierte Gegenüberstellung von Stimuli und Reaktionen, insbesondere auch in der Erfassung und ggf. Ausgabe/Darstellung der zeitlichen Veränderung nach dem Setzen des betreffenden Stimulus, da mit der Erfindung die Reaktionen des Sehapparates auf die unterschiedlichen Stimuli in objektiver Weise und mit hoher zeitlicher Auflösung erfasst werden können.

Das Sehtestgerät ermöglicht beispielsweise auch eine Live-Steuerung und - Verfolgung von Akkommodation, Konvergenz und/oder der Blickrichtung (Fixationspunkt) beider Augen am Bildschirm.

Das Sehtestgerät bietet z. B. auch die Möglichkeit der Aufzeichnung der Messergebnisse, die Gegenüberstellung zu früheren Messungen oder die Gegenüberstellung zu Messwerten von anderen Patienten, vielfältige Visualisierungsmöglichkeiten (Karten, Diagramme, Kennzahlen) einschließlich neuartiger Visualisierungen für eine "Diagnose mit einem Blick".

Das Sehtestgerät ermöglicht insbesondere auch eine hohe Objektivierung der Untersuchungsverfahren und hohe Reproduzierbarkeit der Untersuchungsbedingungen sowie computergestützt die automatische Durchführung von verschiedenartigen Untersuchungen auf Basis unterschiedlicher Computerprogramme.

Das Sehtestgerät kann in kompakter Weise aufgebaut werden, beispielweise auf Basis ophthalmoskopischer Verfahren für die Messung der Blickrichtungen und der Akkommodation beider Augen bei Einsatz von Infrarottechnik für Beleuchtungs-, Abbildungs- und Messtechnik.

Das Sehtestgerät ermöglicht z. B. auch die Erzeugung von Diagrammen, welche den Zusammenhang zwischen Parametern bzw. Messgrößen visualisieren: Fixationspunktkoordinaten (Stimulus und Messgröße), Konvergenzwinkel (Stimulus und Messgröße), Akkommodation (Stimulus und Messgröße), Zeit, physiologische Parameter des Probanden, Häufigkeit einer Messgröße.

Das Sehtestgerät ermöglicht insbesondere auch computergesteuert durch dynamisch durchgeführte "Aufdeck-" und "Abdecktests" die Feststellung von Tropien und Phorien.

Das Sehtestgerät ermöglicht z. B. ferner eine Fernsteuerung des Untersuchungsablaufs und eine telemedizinische Fernbefundung.

Für die Anwendung in der medizinischen Praxis ergeben sich durch die Erfindung neue Möglichkeiten und vielfältige Vorteile:
- Das Sehtestgerät ermöglicht die schnelle Aufdeckung von strabologischen Erkrankungen (Tropien, Phorien, Fixationsprobleme, Mikrostrabismus etc.).
- Die technische Aufzeichnung der Untersuchungsergebnisse ermöglicht eine umfassende Erfassung von Krankheitsbildern, die Analyse von zeitlichen Krankheitsverläufen, die Visualisierung der Messergebnisse in übersichtlichen Zusammenfassungen (Karten, Diagramme, Kennzahlen) sowie die Speicherung der Ergebnisse in Datenbanken zwecks übergreifender Auswertungen.
- Ein herausragender Vorteil des Sehgerätes ist die Möglichkeit, asthenopische Beschwerden gezielt untersuchen zu können. Asthenopische Beschwerden treten bei Abweichungen der Korrespondenz zwischen Konvergenz und Akkommodation auf, beispielsweise auch bei der Wahrnehmung virtueller dreidimensionaler Bilder, die durch die neuen digitalen Medien (3D-Kino, 3D-Fernsehen, Computerspiele) verstärkt dem Auge angeboten werden.
- Das Sehtestgerät ermöglicht auf Basis der flexiblen Stimulation der motorischen Funktionen der Augen und der damit verbunden neurologischen und neurophysiologischen Prozesse Spezialuntersuchungen, die mit den bisher zur Verfügung stehenden Geräten und Untersuchungsverfahren nicht möglich sind.
- Die bei ophthalmologischen Untersuchungen häufig auftretende Instrumentenakkommodation wird bei dem Sehtestgerät durch die kontrollierte und unabhängig voneinander ausführbare Stimulation von Konvergenz und Akkommodation unter gleichzeitiger Messung und Visualisierung der Reaktionen des Sehapparates vermieden.
- Mit dem Sehtestgerät kann eine Vielzahl unterschiedlicher ophthalmologischer Untersuchungsverfahren mit einer einzigen Apparatur realisiert werden.
- Die einfache und automatisierbare Ausführung strabologischer Untersuchungen ermöglicht Routine-Voruntersuchungen in Praxen ohne eigene Orthoptistinnen nach dem Vorbild des Einsatzes von Autorefraktometern. Auch für Reihenuntersuchungen in Schulen und Kindergärten kann das erfindungsgemäße Sehtestgerät vorteilhaft eingesetzt werden.
- Ebenso ist ein Einsatz des Sehtestgerätes in Therapieeinrichtungen und für eine Therapienachverfolgung von Bedeutung.
- Erweiterte Einsatzmöglichkeiten ergeben sich aufgrund der Objektivität der Untersuchungsverfahren im Bereich der Telemedizin.
- Aufgrund der hohen Objektivität und Reproduzierbarkeit der Untersuchungsbedingungen erschließen sich neue Möglichkeiten für die medizinische Forschung.

Hinsichtlich eines Bilderzeugungsmoduls, eines Abbildungsmoduls, einer Akkommodationsmesseinrichtung und/oder einer Blickrichtungsmesseinrichtung ist vor dem jeweiligen Auge bzw. Okular ein gemeinsamer Strahlengang vorgesehen.

Zweckmäßigerweise ist eine Kamera, vorzugsweise digitale Kamera, vorgesehen, mittels der das jeweilige Auge oder ein Teil davon, vorzugsweise der Fundus des Auges oder ein Teil davon laufend abgetastet wird und hierdurch eine fortlaufende Serie von Daten erzeugt wird, mittels welcher die Akkommodation sowie die Blickrichtung des jeweiligen Auges durch einen Computer bzw. ein gemeinsames Steuerungs- und Auswertemodul ermittelt werden kann. Unter dem Begriff Kamera ist ein Bildsensor zur Erzeugung von Bilddaten zu verstehen. Hierunter fallen z. B. auch Einrichtungen zur Erzeugung von Bilddaten unter Verwendung von Laserlicht.

Zweckmäßigerweise ist für jeden Strahlengang, d. h. für jedes Auge eine eigene Kamera vorgesehen, sodass die Untersuchung jedes der beiden Augen zeitgleich erfolgen kann.

Zweckmäßigerweise werden in den Kameras Bilddaten erzeugt, die in eine gemeinsame Auswertung, vorzugsweise als kontinuierlicher Datenstrom, einfließen können.

Vorzugsweise ist für die Akkommodationsmesseinrichtung (Refraktometer) sowie für die Blickrichtungsmesseinrichtung (Eye-Tracker) eine Kamera gemeinsam vorgesehen, was den apparativen Aufbau sowie die Vorkehrungen zur Auswertung des Bildmaterials vereinfacht.

Dadurch, dass für die Akkommodationsmesseinrichtung sowie getrennt für die Blickrichtungsmesseinrichtung jeweils ein eigenständiges Beleuchtungsmodul, z. B. IR-Beleuchtungsmodul, welches vorzugsweise mit Hilfe jeweils einer IR-LED realisiert wird, vorgesehen ist, besteht die Möglichkeit, unterschiedliche Abbildungs- bzw. Beleuchtungsflächen insbesondere auf dem Fundus (Augenhintergrund) des betreffenden Auges zu erzeugen, die für die Erfassung einerseits der Akkommodation sowie andererseits der Blickrichtung dienen.

Zweckmäßigerweise erfolgt eine photographische Messwerterfassung durch die Akkommodationsmesseinrichtung sowie durch die Blickrichtungsmesseinrichtung, alternativ vorzugsweise in sehr kurzen Zeitintervallen. Hierdurch lässt sich bezüglich der gewünschten Messwerte eine sehr hohe Datendichte nahezu zeitgleich erzielen.

Die Erfassung der Blickrichtung erfolgt ophthalmoskopisch auf der Grundlage des Fundus (Augenhintergrund) des betreffenden Auges. Das hat den Vorteil, dass die absolute Blickrichtung ohne Kalibrierung festgestellt werden kann. Daraus resultiert der Vorteil, dass die Bewegung der Makula direkt erfasst werden kann und ein eventuell vorhandener Mikrostrabismus hierbei zusätzlich erkennbar wird.

Erfindungsgemäß kann die Messung der Akkommodation des jeweiligen Auges sowie die Messung der Blickrichtung des jeweiligen Auges fortwährend gleichzeitig oder fortlaufend alternierend durchgeführt werden. In jedem Fall wird ein zeitlicher Verlauf an Messdaten des menschlichen Sehvermögens auf einen speziellen Stimulus hin geschaffen.

Die Strahlengänge zur Erzeugung der Stimuli mittels Testfiguren durch das Bilderzeugungsmodul und die Strahlengänge zur Messung der Reaktion der Augen auf die Stimuli erfolgt insbesondere bei unterschiedlichen Wellenlängen.

Zur Vereinigung der Strahlengänge unterschiedlicher Wellenlänge kann ein dichroitischer Spiegel vorgesehen sein.

Eine Maske im Beleuchtungsstrahlengang der Akkommodationsmesseinrichtung dient dazu, ein Testmuster auf dem Augenfundus abzubilden, wodurch aus der Bildinformation in Verbindung mit der aktuellen Einstellung des optischen Elementes im Strahlengang die Akkommodation und Refraktion direkt vom Computer berechnet werden kann.

Ein zweiter Beleuchtungsstrahlengang dient insbesondere dazu, unabhängig vom Abbild des Testmusters einen bestimmten Bereich des Auges, z. B. den Fundus, auszuleuchten und anhand von im ausgeleuchteten Bereich vorhandenen Strukturen, z. B. Netzhautstrukturen, die Fixationsrichtung d.h. die Blickrichtung festzustellen.

Das gemeinsame Steuerungs- und Auswertemodul kann eine Logik umfassen oder mit einem Computer ausgestattet oder verbunden sein. Es umfasst Ausgabegeräten zur Speicherung, Visualisierung und/oder Dokumentation der Messergebnisse. Vom gemeinsamen Steuerungs- und Auswertemodul bzw. vom Computer aus erfolgt in Verbindung mit einer geeigneten Ansteuerelektronik die Erzeugung der Stimuli. Hierbei kann z. B. auch die Möglichkeit vorgesehen sein, eine manuelle Auswahl und Steuerung der Stimuli direkt durch den Untersucher vorzunehmen. Alternativ oder zusätzlich können dem Computer auch feste Messprozeduren einprogrammiert werden, sodass Untersuchungen automatisiert durchgeführt werden können.

Zur Erzeugung des Testbilds kann die Vorrichtung ein mit dem gemeinsamen Steuerungs- und Auswertemodul bzw. dem Computer in Datenverbindung stehendes Display des Bilderzeugungsmoduls umfassen, auf dem beliebige Testbilder erzeugt werden und durch einen optischen Strahlengang auf die Netzhaut des Probanden projiziert werden können.

Es ist zweckmäßig, wenn gemäß der vorliegenden Erfindung die Stimuli für Augenbewegungen, Fixation, Konvergenz unabhängig von den Stimuli für die Akkommodation erzeugt werden, sodass unterschiedliche AC/A-Stimuli und/oder CA/C-Stimuli generiert werden können.

Zweckmäßiger kann mit dem erfindungsgemäßen Verfahren auch eine 3D Stimulation des menschlichen Auges vorgenommen werden. Hierzu sind insbesondere unterschiedliche Fixationspunkte generierbar, wodurch die 3D Sehfähigkeit bzw. Tiefensehfähigkeit und zwar bezogen auf das jeweilige Auge getestet werden kann. Hierbei können beliebig unterschiedliche Akkommodations- und/oder Konvergenzreize geschaffen werden.

Es ist vorteilhaft, wenn der Abstand der beiden Augen zueinander, vorzugsweise durch Änderung und Registrierung des Abstands der Strahlengänge zueinander, ermittelt wird.

Vorzugsweise wird die Stimulation der Augenbewegungen, Fixation, Akkommodation und/oder Konvergenz automatisch nach einem vorgegebenen Programm durchgeführt.

Dadurch, dass das Testbild mindestens ein, vorzugsweise eine Mehrzahl von Objekten, z.B. geometrische Figuren wie Kreisen oder dergleichen, aufweist, das bzw. die im Randbereich der Makula, d.h. des Bereichs der größten Häufigkeit von Sehzellen abgebildet wird bzw. werden, kann das erfindungsgemäße Verfahren auch bei Patienten mit einem Makulaleiden (z.B. bei einer Makuladegeneration) vorteilhaft angewendet werden. Patienten mit fehlendem, foveolarem Sehen aber intaktem, parafoveolaren Sehen können die im Randbereich der Makula positionierten Objekte erkennen und die Mitte der Lage der Objekte anfixieren. Der Anwendungsbereich des erfindungsgemäßen Verfahrens wird hierdurch deutlich erweitert.

Das erfindungsgemäße Verfahren erlaubt es insbesondere auch, dass die Stimuli für Akkomodation und Konvergenz gleichzeitig generiert und/oder vorzugsweise auch unabhängig voneinander variiert werden. Bei einer kombinierten Anwendung der beiden vorher genannten Stimuli können diese zur Simulation eines natürlichen Stimulus durch das erfindungsgemäße Verfahren aufeinander abgestimmt werden.

### Beschreibung der Erfindung anhand eines Ausführungsbeispiels

Die Erfindung wird nachfolgend an einem Ausführungsbeispiel beschrieben. Dazu zeigen:
- Fig. 1: eine Prinzipdarstellung der Vorrichtung zur Analyse von Konvergenz und Akkommodation der Augen gemäß der Erfindung;
- Fig. 2: die Prinzipdarstellung eines möglichen Ausführungsbeispiels zur Durchführung des erfindungsgemäßen Verfahrens;
- Fig. 3: ein Beispiel für die Erfassung und Visualisierung von "Einschwingvorgängen", die beim Aufdecktest (Einschalten eines Stimulus für die Konvergenz der Augen) auftreten;
- Fig. 4: eine beispielhafte Darstellung der Visualisierung des Einstellverhaltens der Augen auf Akkommodations- sowie Konvergenzstimuli;
- Fig. 5: das Hystereseverhalten der Augen beim Setzen unterschiedlicher Akkommodations- sowie Konvergenzstimuli;
- Fig. 6: Untersuchungsbeispiele mit natürlichem Stimulus,
- Fig. 7: Untersuchungsbeispiele mit 3D-Kino-Stimulus,
- Fig. 8: eine Darstellung verschiedener Möglichkeiten der Anordnung photographisch zu erfassender Abbildungsbereiche auf der Netzhaut, sowie
- Fig. 9: eine stark vereinfachte, schematische Darstellung der Erzeugung eines Testbilds mit beispielsweise vier Objekten, die im Außenbereich der Makula positioniert sind.

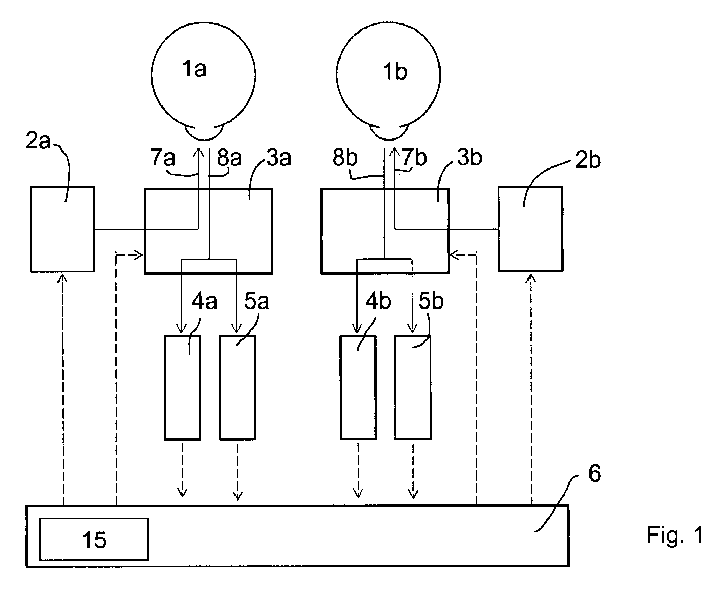

Ein Ausführungsbeispiel der erfindungsgemäßen Vorrichtung zur Analyse von Konvergenz und Akkommodation der Augen ist schematisch in Fig. 1 dargestellt. Es umfasst Bilderzeugungsmodule 2a und 2b, Abbildungsmodule 3a und 3b, Akkommodationsmesseinrichtungen 4a und 4b und Blickrichtungsmesseinrichtungen (Eye-tracker) 5a und 5b, die jeweils baugleich entweder dem rechten oder dem linken Auge 1a bzw. 1b zugeordnet sind. Ein Steuerungs- und Auswertemodul 6 mit Computer 15 dient der Steuerung der Komponenten sowie der Erfassung und Auswertung der Messwerte.

Die Komponenten sind für jedes Auge so angeordnet, dass durch sie jeweils zwei Strahlengänge realisiert werden. Mittels der Komponenten im Strahlengang 7a bzw. 7b werden Stimuli für die Konvergenz und Akkommodation der Augen generiert. Die Reaktionen des Sehapparates auf die Stimuli werden mittels der Komponenten im Strahlengang 8a bzw. 8b beobachtet, gemessen und erfasst.

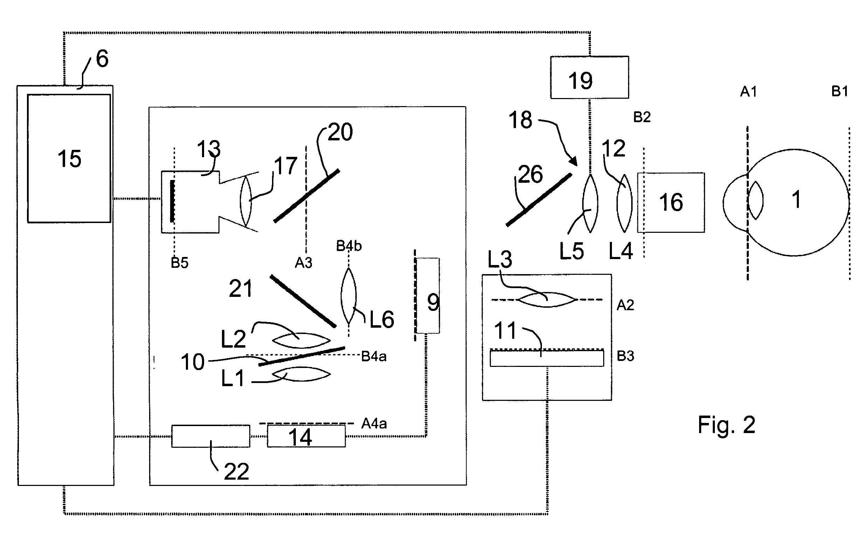

Anhand der Prinzipdarstellung in Fig. 2 wird ein Ausführungsbeispiel des erfindungsgemäßen Sehtestgerätes zur Analyse von Konvergenz und Akkommodation der Augen und für die Durchführung des erfindungsgemäßen Verfahrens erläutert. In Fig. 2 sind außer einem Computer 15 als Hauptelement des Steuerungs- und Auswertemoduls 6 alle Komponenten für die Stimulation des Sehapparates und zur Erfassung von Messwerten nur für ein Auge wiedergegeben. Zusätzlich zu den dargestellten Komponenten sind die gleichen Komponenten für das zweite Auge vorhanden und in analoger Weise mit dem Steuerungs- und Auswertemodul 6 mit dem Computer 15 gekoppelt.

Vom Computer 15 werden zur Stimulation des Sehapparates auf dem Display 11 beliebige Testbilder bzw. Testfiguren erzeugt. Der Computer 15 stellt dem Untersucher verschiedene Standardtestfiguren zur Verfügung, aus denen er die Testfigur auswählt, die für die gewünschte Untersuchung geeignet ist. Durch Anzeigen eines Lichtpunktes kann die Fixationsfähigkeit überprüft werden, die Konvergenz durch Anzeigen einer Fixationsmarke getrennt für beide Augen und die Stereopsis durch Anzeigen eines Stereobild-Paares.

Die Testfiguren für das rechte und das linke Auge werden jeweils auf gesonderten Displays 11 in unmittelbarer Nähe zum Auge 1 angezeigt, wobei das erste Auge die Testfigur für das zweite Auge nicht sehen kann und umgekehrt.

Das Display 11 befindet sich in der Bildebene B3, die durch die Linse L3 auf die Bildebene B2 abgebildet wird. Ein Okular 16 zwischen Auge und Display 11 sorgt dafür, dass das Displaybild in die Ferne abgebildet wird. Durch das Okular 16 betrachtet, scheinen die Testfiguren auf dem Display 11 dann in sehr weiter Ferne zu sein. Zur Reduktion von Reflexionen sind die Linsen des Okulars 16 mit einer (in der Zeichnung nicht dargestellten) Antireflexionsbeschichtung versehen. Eine Feldlinse 12 sorgt dafür, dass die Apertur der Projektionslinse L3 und die Apertur des Kameraobjektivs 17 optimal auf die Pupille des Auges 1 abgebildet werden.

Zur Stimulation der Akkommodation ist im Strahlengang zusätzlich zum Okular 16 eine Fokussiereinheit 18 angebracht. Als Bestandteil der Fokussiereinheit 18 wird beispielsweise eine Gel-Linse L5 verwendet. Die Einstellung der jeweils benötigten Brechkraft der Linse wird durch die Ansteuereinheit 19 realisiert. Die Linse L5 kann auch zum Ausgleich sphärischer Fehlsichtigkeiten verwendet werden. Bei Astigmatismus ist eine zusätzliche Korrekturlinse erforderlich.

Die Fokussiereinheiten 18 erlauben es, für beide Augen unabhängig voneinander die Fokuslage der Testfiguren der Displays 11 zu variieren. So können die von den Displays 11 angezeigten Testfiguren vom Fernpunkt der Akkommodation bis zum Nahpunkt und darüber hinaus bei gleichzeitiger Variation des Konvergenzwinkels verschoben werden.

Neben der stereoskopischen Entfernungseinstellung der Augen, die durch den Konvergenzwinkel gegeben ist und durch die getrennt für beide Augen erzeugten Testfiguren herbeigeführt wird (s.o.), lässt sich mittels der Fokussiereinheit 18 zusätzlich ein Stimulus für die Entfernungswahrnehmung auf Basis der Fokuslage der Augen erzeugen. Die beiden Parameter "stereoskopische Entfernung" und "Fokusentfernung" sind unabhängig voneinander einstellbar und stimulieren die entsprechenden Reaktionen Konvergenz und Akkommodation.

Die Messung der Fixation erfolgt für beide Augen separat mittels einer Blickrichtungsmesseinrichtung 5. Die Blickrichtungsmesseinrichtung 5 ermittelt die Blickrichtung eines Auges z.B. durch Bestimmung des Hornhaut-Lichtreflexes und/oder des Netzhautbildes. Anhand der Messung der Blickrichtungen beider Augen wird durch den Computer 15 der Konvergenzwinkel berechnet. Aus dem Konvergenzwinkel lässt sich weiterhin die stereoskopische Entfernung ermitteln, auf welche der Proband seine Augen eingestellt hat. Somit kann überprüft werden, ob der Proband physiologisch und neurologisch korrekt auf den Stimulus reagiert. Abweichungen des Verhältnisses zwischen Akkommodation und Konvergenz können zeitaufgelöst ermittelt und einer Auswertung zugänglich gemacht werden.

Sofern im Bedarfsfall die Akkommodation sowie der Fixationsfähigkeit von nur einem Auge getestet werden soll, es also auf die Ermittlung der Blickrichtung des zweiten Auges zur Konvergenzbestimmung nicht ankommt, muss die erfindungsgemäße Vorrichtung nicht binokular ausgebildet sein. Eine solche Vorrichtung hätte somit lediglich einen der jeweiligen in Fig. 1 dargestellten Strahlengänge z.B. 7a sowie. 8a, nicht jedoch 7b sowie 8b.

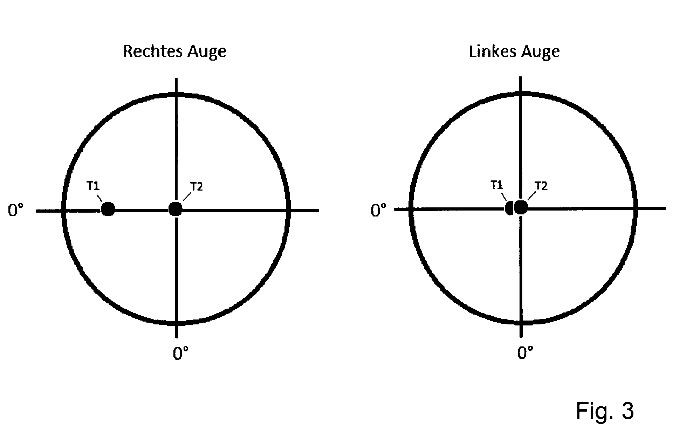

Einschwingvorgänge des Konvergenzwinkels und der Akkommodation können zeitaufgelöst aufgezeichnet und, falls erforderlich, auch an Ausgabegeräte wie z. B. einem Bildschirm oder dgl. ausgegeben werden. Beispielsweise zeigt Fig. 3 den zeitlichen Verlauf der Stimuli Dₛ und l/d_{S} für Akkommodation bzw. Konvergenz. Außerdem ist der zeitliche Verlauf der gemessenen Akkommodation D_{R} bzw. 1/d_{R}, d.h. die Reaktion auf die Stimuli, aufgetragen. Man erkennt eine zeitliche Verzögerung Δt_{A} bzw. Δt_{K} zwischen Stimulus und Reaktion. Als vertikale Achse ist zur besseren Ablesbarkeit der reziproke Wert in der Einheit Meter aufgetragen. Dieser Wert entspricht der simulierten Entfernung in welche die Testfigur projiziert wird bzw. der Entfernung auf welche der Proband seine Augen einstellt. Ebenso kann in einer in den Figuren nicht dargestellten "Live"-Darstellung z. B. der zeitliche Verlauf der Blickwinkeländerung an einem Computerdisplay dargestellt werden.

Für die Funktionsweise des Gesamtverfahrens muss die Methode, nach welcher die Blickrichtungsmesseinrichtung 5 funktioniert, keine spezielle sein. Vorteilhaft ist jedoch eine Realisierung der Blickrichtungsmesseinrichtung 5 durch je eine ophthalmoskopisch arbeitende Messeinrichtung für beide Augen, welche z.B. mit Infrarotlicht (IR) den Fundus der Augen aufnimmt. Analog zu einer Änderung der Blickrichtung (=Rotation des Auges) verschiebt sich der Inhalt des Fundusbildes. Aus der Position bestimmter Netzhautstrukturen (insbesondere der Sehgrube) lässt sich somit auf die Blickrichtung des Probanden schließen. Ein weiterer Vorteil dieses Verfahrens ist die Möglichkeit einer absoluten Bestimmung der Blickrichtung. Blickrichtungsmesseinrichtungen, welche z.B. Reflexionen an der Cornea auswerten, können gut relative Bewegungen feststellen, sie sind jedoch ungenau hinsichtlich der absoluten Blickrichtung. In dem Fall wäre eine Kalibrierung erforderlich. Durch das ophthalmoskopische Verfahren hingegen lässt sich direkt die Makula verfolgen, wodurch auch ein evtl. vorhandener Mikrostrabismus erkannt werden kann.

Zur Messeinrichtung eines Auges 1 gehört eine Digital-Kamera 13 einschließlich Objektiv 17, das Okular 16 in Verbindung mit der Feldlinse L4, der Spiegel 20 und eine Infrarot-Beleuchtung für den Augenhintergrund. Die Beleuchtung umfasst zwei Strahlengänge, welche über den Strahlteiler 21 vereinigt und zum Spiegel 20 gelenkt werden.

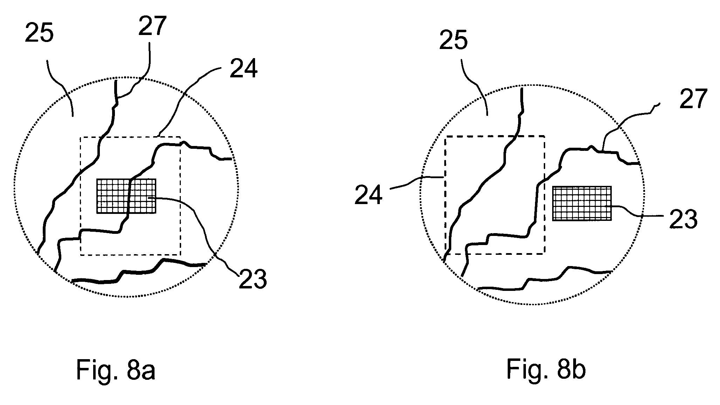

Einer dieser Strahlengänge wird durch ein Beleuchtungsmodul 14, z. B. eine IR-LED erzeugt. Die konvexen Linsen L1 und L2 bilden die IR-LED auf die Aperturebene A3 ab. Die Linse L2 bildet in Verbindung mit der Linse L5 und der Linse L4 außerdem die Bildebene B4a auf die Bildebene B2 ab. Eine Maske 10, welche zur Erzeugung eines Testmusters 23 (vgl. Fig. 8a sowie 8b) auf dem Fundus (Netzhaut 25) dient, schneidet die Bildebene B4a. Der Schnittwinkel zwischen B4a und 10 ist so eingestellt, dass beide Ebenen nur leicht von der Parallelität abweichen. Durch die leichte Schräglage der Maske 10 bezüglich der Bildebene A4a wird erreicht, dass die Maske auf dem Fundus mit örtlich variabler Fokuslage abgebildet wird. Dies registriert der Computer 15 des Steuerungs- und Auswertemoduls 6 über die Kamera 13 und kann aus den Bildinformationen in Verbindung mit der aktuellen Einstellung der Linse L5 die Akkommodation und Refraktion berechnen.

Der andere Beleuchtungsstrahlengang wird durch das Beleuchtungsmodul 9, ebenfalls z.B. ein IR-LED, erzeugt. Im Gegensatz zum Strahlengang für die Bestimmung von Akkommodation wird keine Maske auf den Fundus projiziert sondern eine homogen leuchtende Fläche. Statt der zwei Linsen L1 und L2 wird nur eine Linse L6 verwendet. Mit dieser Beleuchtung wird der gesamte Fundus innerhalb des Beobachtungsbereiches ausgeleuchtet, sodass die Netzhautstrukturen zur Feststellung der Fixationsrichtung verfolgt werden können.

Zur quasi-simultanen Messung der Akkommodation und Fixationsrichtung kann der Computer 15 die LED-Treiber 22 so ansteuern, dass pro aufgenommenem Bild der Kamera 13 abwechselnd das Beleuchtungsmodul 14 bzw. das Beleuchtungsmodul 9 eingeschaltet ist. Dadurch können in schneller Folge abwechselnd Akkommodation und Fixation bestimmt werden.

Alternativ können beide Beleuchtungsmodule 14 und 9 auch dauerhaft eingeschaltet sein, wobei die Maske 10 in einer Netzhautregion abgebildet wird, die zur Verfolgung von Netzhautstrukturen zur Fixationsrichtungsbestimmung nicht nötig ist. In diesem Fall werden Akkommodation und Konvergenz tatsächlich simultan gemessen.

Der Spiegel 20 dient der Trennung der Strahlengänge in einen Beleuchtungsstrahlengang und einen Beobachtungsstrahlengang.

Die Akkommodation wird mit der gleichen Technologie bestimmt, welche auch für die Refraktometrie verwendet werden kann. Die Akkommodation zeigt sich beim normalsichtigen Auge dann in Form einer scheinbaren Kurzsichtigkeit, da ein Autorefraktometer ein deakkommodiertes Auge voraussetzt. Es handelt sich hierbei um ein Standardmessverfahren, das für jedes Auge separat angewendet wird. Die Akkommodation kann für jedes Auge separat ermittelt werden. Außerdem lässt sich eine ggf. vorhandene Fehlsichtigkeit bestimmen. Diese Fehlsichtigkeit wird vom Computer 15 berücksichtigt und über die Stimulus-Einheit ausgeglichen. Das ist jedoch nur für sphärische Fehlsichtigkeiten möglich. Bei Astigmatismus wird die Fehlsichtigkeit durch Korrekturgläser ausgeglichen. Ggf. kann auch eine entsprechende automatische Korrektureinheit eingesetzt werden.

Sowohl zur Messung der Fixation/Konvergenz als auch der Akkommodation werden ophthalmoskopische Messverfahren eingesetzt, die teilweise dieselben Bauelemente verwenden.

Die Vereinigung der Strahlengänge zur Erzeugung der Stimuli mittels Testfiguren und zur Messung der Reaktion der Augen auf die Stimuli mittels der ophthalmoskopischen Messverfahren erfolgt vermöge der unterschiedlichen Wellenlängen in beiden Strahlengänge durch einen dichroitischen Spiegel 26.

Der Computer 15 ist mit (in der Fig. 2 nicht dargestellten) Ausgabegeräten zur Speicherung, Visualisierung und/oder Dokumentation der Messergebnisse ausgerüstet oder verbindbar. Vom Computer aus erfolgt in Verbindung mit geeigneter Ansteuerelektronik die Erzeugung der Stimuli. Hierbei ist z. B. auch eine manuelle Auswahl und Steuerung der Stimuli direkt durch den Untersucher möglich. Alternativ können dem Computer 15 auch feste Messprozeduren einprogrammiert werden, sodass Untersuchungen automatisiert durchgeführt werden können.

Bei einer manuellen Untersuchung interpretiert der Untersucher die Reaktionen des Probanden und variiert ggf. entsprechend dem Untersuchungsziel die Stimuli, um weitere Erkenntnisse zu gewinnen.

Bei einer automatischen Untersuchung kann eine Variation der Stimuli automatisch statt finden. Hierbei kann der Verlauf der Stimulation auch in Abhängigkeit der Reaktionen des Probanden berechnet werden.

Die Messergebnisse der automatischen Stimulus-Präsentation werden aufgezeichnet und geeignet automatisch ausgewertet. Die Ergebnisse der Reaktionsmessungen werden entweder in Echtzeit (Live-Übertragung) visualisiert, für den Untersucher graphisch dargestellt oder automatisch für eine spätere Auswertung aufgezeichnet. Mittels geeigneter Algorithmen werden aus den Fundusbildern die Blickrichtungen und Fixationspunkte bestimmt. Durch Auswertung der auf dem Fundus abgebildeten Maske wird die Akkommodation ermittelt.

Die Software für das Instrument kann je nach Anwendungsfall ausgewählt werden, wodurch die gleiche Hardware für viele verschiedene Anwendungsfälle eingesetzt werden kann. Neben der allgemeinen Steuerung des Systems können z. B. auch durch Algorithmen, die beim gesunden Auge erwarteten Reaktionen vorausberechnet und den tatsächlichen Reaktionen gegenübergestellt werden. Aus den ermittelten Werten für die akkommodative Konvergenz und die Akkommodation werden insbesondere der AC/A-Quotient und/oder der CA/C-Quotient (Konvergenz-Akkommodation) ermittelt. Schließlich kann z.B. die Differenz von Soll- und Ist-Werten und vieles mehr visualisiert werden. Der Abstand der beiden Augen zueinander wird jeweils vorher gemessen und am Gerät eingestellt.

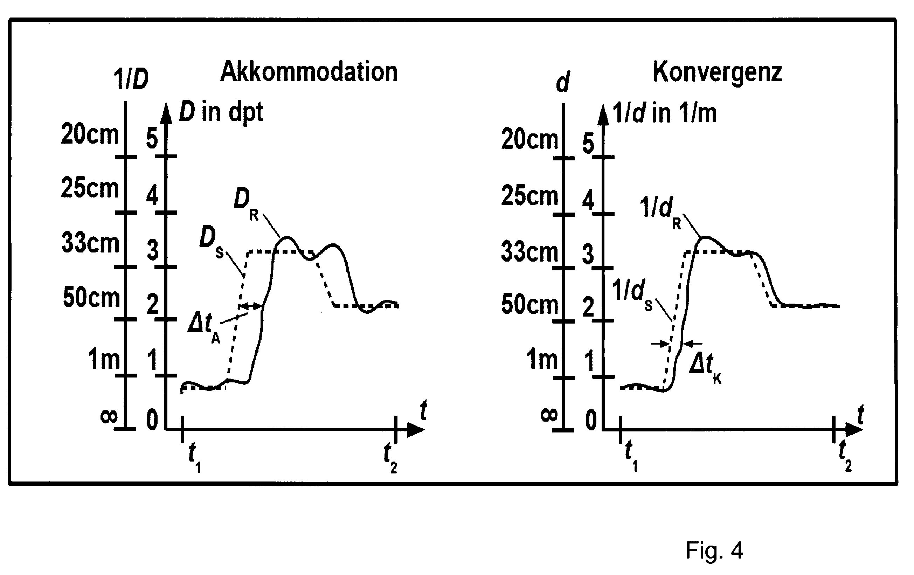

Ein Beispiel für die Gegenüberstellung von Soll- und Istwerten von Akkommodation sowie Konvergenz unter Einbeziehung des zeitlichen Verlaufs (Dynamik) zeigt Fig. 4. In einem Zeitbereich von t₁ bis t₂ werden fortlaufend Stimuli (gestrichelte Linie) für Akkommodation- und Konvergenz erzeugt. Hierbei können, wie in Fig. 4 dargestellt, die Stimuli für Akkommodation und Konvergenz im Sinne eines konstanten AC/A-Wertes korrespondieren. Dies muss aber nicht sein. Gleichzeitig werden die Reaktionen der Augen für Konvergenz und Akkommodation erfasst und in der Grafik wiedergegeben (durchgezogene Linie).

Bei der Darstellung gemäß Fig. 4 ist der Akkommodationswert für nur ein Auge dargestellt, was bei solchen Probanden sinnvoll ist, bei denen davon auszugehen ist, dass sich linkes und rechtes Auge ähnlich verhalten. Die Darstellung nach Fig. 4 zeigt, wie sich das menschliche Auge bei einem besonders vorgegebenen Stimulus-Relief, wie z.B. der Akkommodation oder der Konvergenz, verhält. Die in der Fig. 4 dargestellten Graphen ermöglichen ein zeitlich hoch aufgelöstes Aufzeigen des Einstellverhaltens der Augen auf unterschiedliche Entfernungen, d.h. das Aufzeigen des dynamischen Verhaltens der Akkommodation und der Konvergenz. Ferner kann zusätzlich auch die Reaktionsverzögerung für Akkommodation Δt_{A} sowie für die Konvergenz Δt_{K} bestimmt werden. Auch kann das Einschwingverhalten der neurologischen Regelkreise (z.B. die Naheinstellungstrias) in der Graphik in Form der wellenartigen Überschwinger gut erkannt werden.

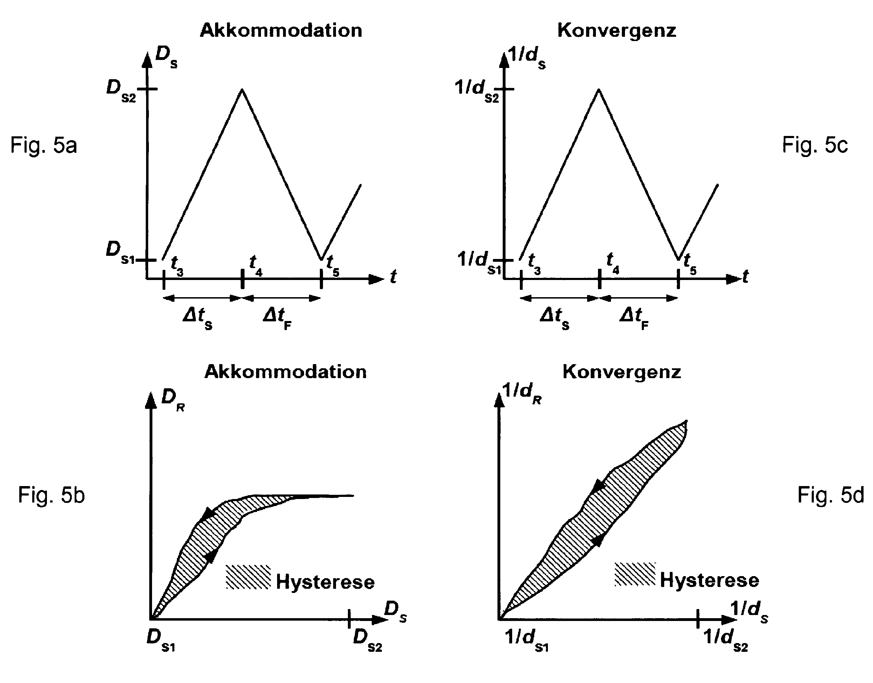

Die Fig. 5 zeigt das Hystereseverhalten der Augen beim Setzen eines Stimulus für die Akkommodation D_{S2} sowie für die Konvergenz 1/dₛ und nachträglicher Rückführung des jeweiligen Stimulus auf den Ausgangszustand. Die Stimuli für Akkommodation bzw. Konvergenz werden über einen Zeitraum Δtₛ variiert. Wie in den Fig. 5a sowie 5c dargestellt, ist der Zusammenhang idealerweise linear. Je nach gewünschtem AC/A-Wert ergeben sich unterschiedliche Steigungen und damit auch die unterschiedlichen Grenzen für D_{S1} sowie D_{S2} bzw. 1/ds₁ und 1/ds₂, wobei es sich bei dem Wert D um die Akkommodationsbrechkraft (Einheit dpt oder Dioptrie) und bei 1/D um den Akkommodationsabstand handelt. 1/d korrespondiert mit D und 1/D mit d. In der Fig. 4 sind die Achsen der Graphen so gewählt, dass D und 1/d auf der jeweiligen Achse linear aufgetragen sind.

Wie in Fig. 5a sowie 5c dargestellt, ist der Zusammenhang bei der Variation der Akkommodation sowie der Konvergenz über ein Zeitintervall Dtₛ linear. Je nach gewünschtem AC/A-Wert ergeben sich unterschiedliche Steigungen und damit auch unterschiedliche Grenzen für D_{S1} sowie D_{S2} bzw. 1/d_{S1} und 1/d_{S2}. Sofern nur ein Vorgang untersucht werden sollte, so z.B. die Akkommodation, kann der Wert der Konvergenz auch konstant gehalten werden (oder umgekehrt), um nur einen der Vorgänge separat zu untersuchen.

Die Fig. 5b sowie 5d zeigen die Reaktionen des menschlichen Auges auf die Stimuli und werden vorzugsweise auch dem Untersucher angezeigt. Hierbei entsteht im Zeitintervall Δtₛ der aufsteigende Ast der Kurve in den Fig. 5b sowie 5d. Danach werden die Stimuli im Zeitintervall Δt_{F} wieder auf den Anfangswert zurückgefahren, wie dies in Fig. 5a sowie 5c dargestellt ist. Hierbei ergeben sich die in den Fig. 5b sowie 5d dargestellten absteigenden Linienverläufe. Die aufsteigenden und absteigenden Linien können unterschiedlich sein und schließen eine Fläche ein, die ein Hystereseverhalten widerspiegelt. Je größer die Fläche ist, desto ausgeprägter ist das Hystereseverhalten.

Falls gewünscht, kann die Messung auch wiederholt werden, wie dies in den Fig. 5a sowie 5c angedeutet ist.

Das Hystereseverhalten ist aufgrund der Reaktionsverzögerungen Δt_{A} bzw. Δt_{K} zu erwarten. Werden beispielsweise die Intervalle Δt_{S} und Δt_{F} verkleinert, steigt der Einfluss der Reaktionsverzögerungen und die Hysterese wird ausgeprägter. So lässt sich durch das erfindungsgemäße Verfahren ergänzend zu den Graphen gemäß Fig. 4 auch mit den Hysteresekurven das Zeitverhalten der Akkommodation und Konvergenz illustrativ aufzeigen.

Eine weitere wichtige Aussage der Fig. 5 ist die Gegenüberstellung der Stimuli zu den dazugehörigen Reaktionen. Möchte man beispielsweise das statische Verhalten der Akkommodation bzw. der Konvergenz aufzeigen, müssten die Intervalle Δt_{S} und Δt_{F} sehr groß im Vergleich zu Δt_{A} bzw. Δt_{K} gewählt werden. In diesem Fall würde sich der ansteigende Verlauf sowie der abfallende Verlauf überlagern und die Hysteresefläche verschwinden. Wenn nur das statische Verhalten gemessen werden soll, ist somit die Messung über Δt_{S} ausreichend.

Das Steuerungs- und Auswertemodul ermöglicht es, z. B. Diagramme oder Tabellen zu erzeugen, welche Zusammenhänge zwischen folgenden Parametern bzw. Messgrößen vorzugsweise paarweise wie folgt aufzeigen können:
- Fixationspunktkoordinate, horizontal, Stimulus, linkes Auge und/oder
- Fixationspunktkoordinate, vertikal, Stimulus, linkes Auge und/oder
- Fixationspunktkoordinate, horizontal, Messgröße linkes Auge und/oder
- Fixationspunktkoordinate, vertikal, Messgröße, linkes Auge und/oder
- Fixationspunktkoordinate, horizontal, Stimulus, rechtes Auge und/oder
- Fixationspunktkoordinate, vertikal, Stimulus, rechtes Auge und/oder
- Fixationspunktkoordinate, horizontal, Messgröße rechtes Auge und/oder
- Fixationspunktkoordinate, vertikal, Messgröße, rechtes Auge und/oder
- Akkommodation, Stimulus, linkes Auge und/oder
- Akkommodation, Messgröße, linkes Auge und/oder
- Akkommodation, Stimulus, rechtes Auge und/oder
- Akkommodation, Messgröße, rechtes Auge und/oder
- Zeit und/oder
- Physiologische Parameter eines oder einer Probandengruppe (z.B. Alter, Fehlsichtigkeit,...)

Zusätzlich zu den oben genannten Parametern bzw. Messgrößen oder alternativ dazu können auch Größen z. B in den Diagrammen dargestellt werden, welche sich durch eine mathematische Berechnung aus den Messgrößen ergeben, wie insbesondere
- Konvergenzwinkel, Stimulus und/oder
- Konvergenzwinkel, Messgröße und/oder
- Vertikaler Schielwinkel und/oder
- Horizontaler Schielwinkel und/oder
- Häufigkeit einer Größe (zur Histogramm-Erstellung).

Beispielsweise können die oben genannten Größen durch Projektionsverfahren in einem Diagramm dargestellt werden (z.B. insbesondere in Form eines sog. 3D-Diagramms). Beispielsweise können eine oder mehrere Größen und/oder Messwerte durch einen Intensitätswert oder Farbwert visualisiert werden, um dadurch ein Intensitätsdiagramm bzw. Farbwertdiagramm oder eine Kombination aus beiden zu erhalten.

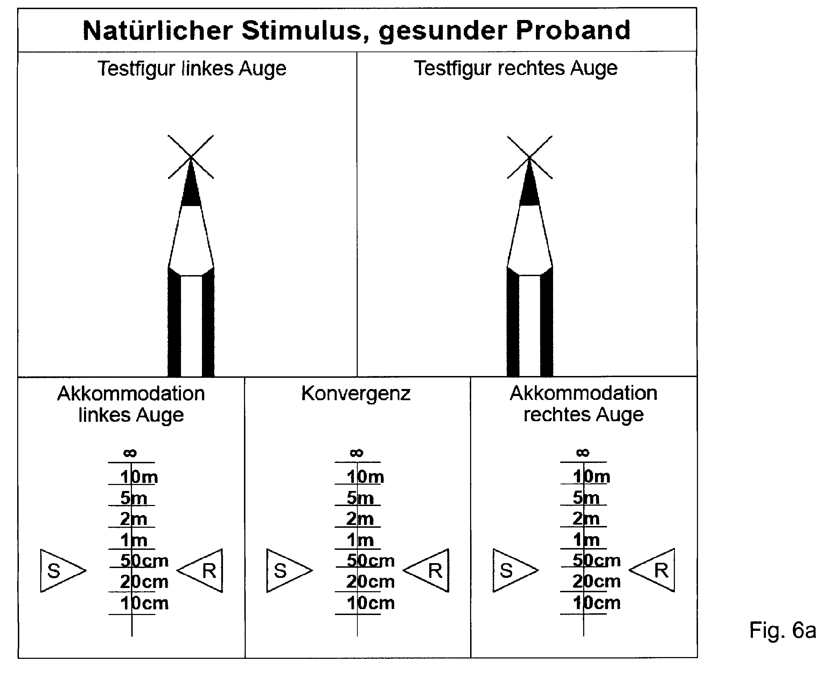

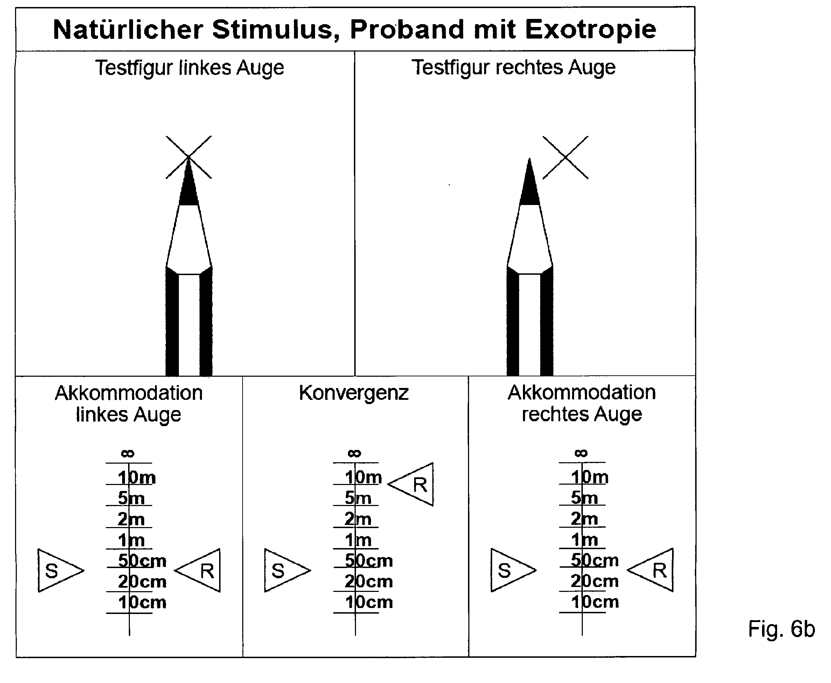

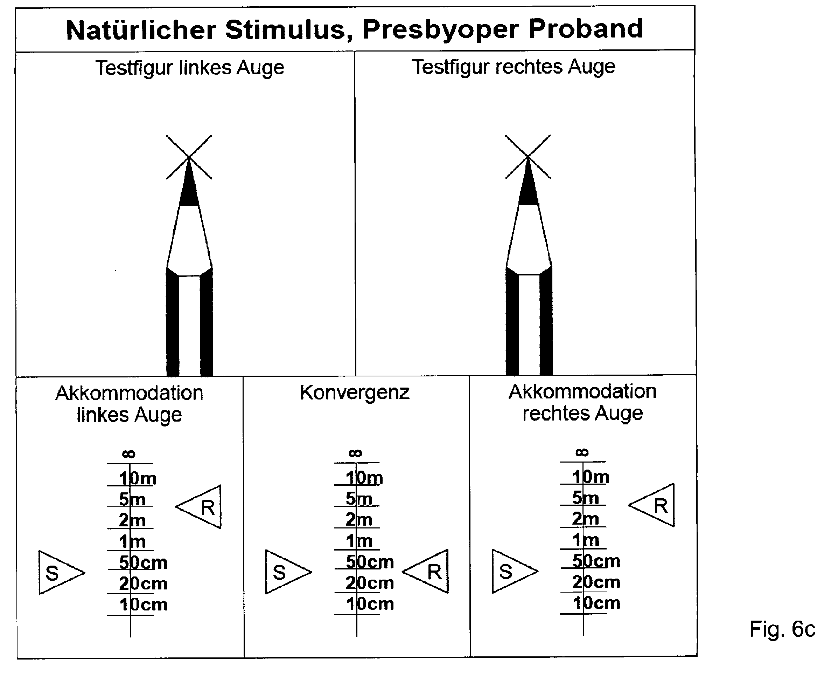

In der Fig. 6 wird beispielhaft gezeigt, wie ein natürlicher Stimulus mittels eines Testbilds (Bleistift) für die Akkommodation des linken Auges, die Akkommodation des rechten Auges sowie die Konvergenz bei einem Probanden gesetzt werden kann. Die Anzeigemarke S steht für den Stimulus, die Anzeigemarke R für die Reaktion des Probanden. Das Kreuz stellt die Anzeigemarke des Fixationspunktes dar, d.h. des Punktes der mittels der Blickrichtungsmesseinrichtung gemessenen Blickrichtung des Probanden bei gesetztem Stimulus.

Fig. 6a zeigt das Ergebnis nach dem Setzen des Stimulus bei einem gesunden Probanden. Bei einem gesunden Probanden stimmen die Reaktionen der Augen mit den gesetzten Stimuli überein.

Bei der Darstellung nach Fig. 6b wird bei einem Probanden eine Exotropie am rechten Auge festgestellt. Die gemessene Blickrichtung des rechten Auges stimmt nicht mit der Spitze der Testfigur (Bleistiftspitze) überein.

Bei der Untersuchung des Probanden der Fig. 6c wird eine Altersfehlsichtigkeit (presbyoper Proband) festgestellt und zwar eine Fehlsichtigkeit, sowohl auf dem rechten als auch auf dem linken Auge. Die Konvergenz d. h. die Augenstellung ist bei diesem Probanden jedoch in Ordnung.

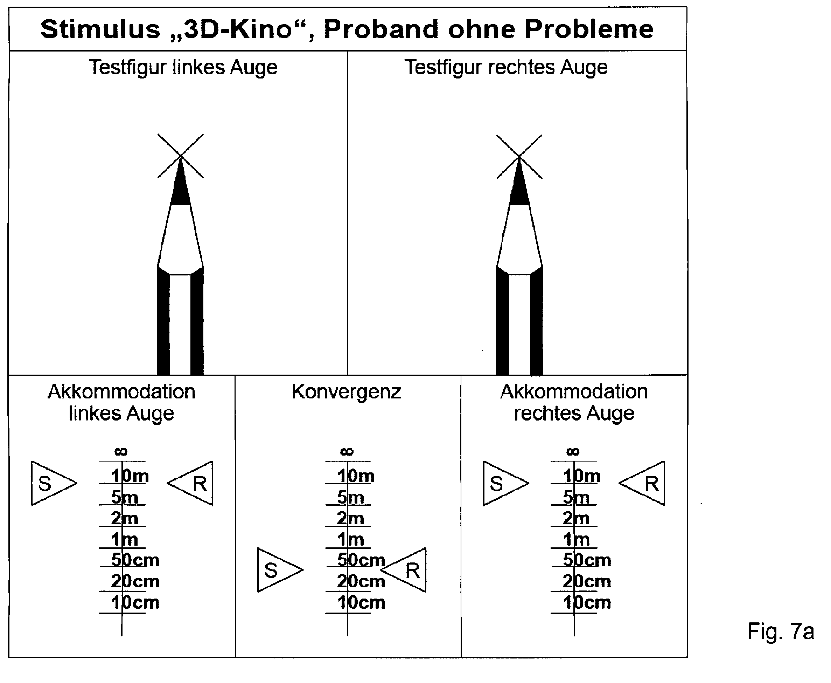

Die Figuren 7a - 7c zeigen einen Stimulus, mit welchem die 3D-Sehfähigkeit eines Probanden getestet werden kann, d.h. es wird getestet, ob ein Proband in der Lage ist ein virtuelles dreidimensionales Bild bzw. Objekt, wie es im "3D-Kino" oder beim "3D-Fernsehen" generiert wird, korrekt zu verarbeiten. Dies ist bei dem erfindungsgemäßen Verfahren durch Variation des AC/A-Quotienten möglich. Bei der Darstellung nach Fig. 7a wird die Konvergenz im Vergleich zur Akkommodation verändert, indem der Stimulus der Konvergenz auf eine geringere Entfernung als der Stimulus der Akkommodation des jeweiligen Auges eingestellt wird. Die Reaktion des Probanden in Bezug auf Akkommodation sowie Konvergenz entspricht dem jeweils vorgegebenen Stimulus. Der Proband hat in Bezug auf die 3D-Sehfähigkeit keine Probleme.

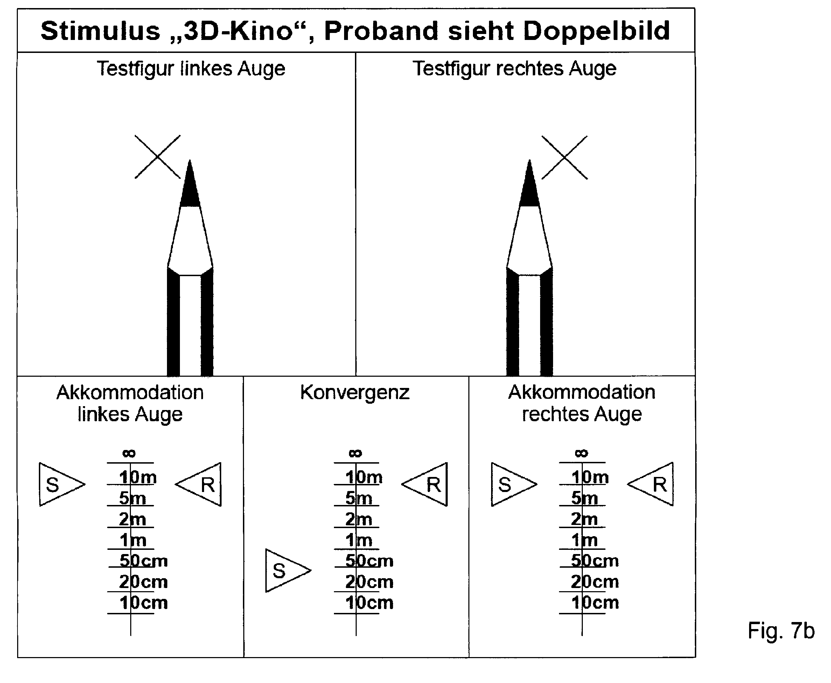

Bei der Darstellung nach Fig. 7b wird demgegenüber eine Abweichung der Reaktion des Stimulus der Konvergenz festgestellt, d.h. die gemessenen Blickrichtungen auf beiden Augen weichen von der tatsächlichen Position (Spitze des Bleistifts) ab. Der Proband sieht ein Doppelbild.

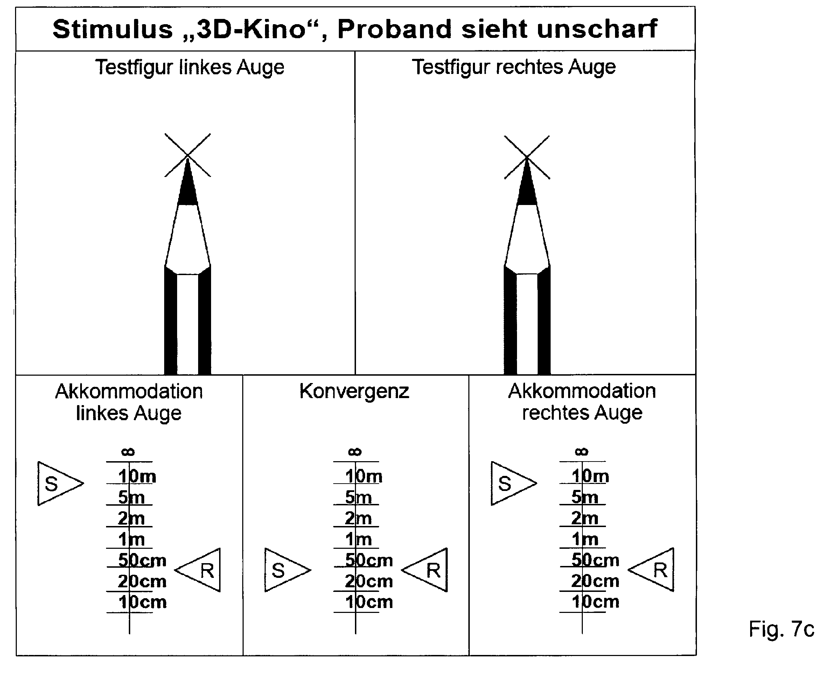

Bei der Darstellung nach Fig. 7c wiederum wird auf beiden Augen eine unpassende Akkommodation festgestellt. Der Proband sieht das Testbild lediglich unscharf, wobei aber die Augenstellung, d.h. Fixationsrichtung (Konvergenz) mit dem Stimulus übereinstimmen.

Bei dem in Fig. 7b oder 7c beispielhaft aufgezeigten Krankheitsbild (asthenopische Beschwerden) kann das erfindungsgemäße Verfahren dazu verwendet werden, beim 3D-Sehen eines 3D-Films oder eines 3D-Bildes die Ermittlung desjenigen Sehabstands bzw. Sehabstandsbereich vorzunehmen, bei dem die Akkommodation und die Konvergenz zumindest im Wesentlichen im Gleichklang stehen und somit keine asthenopische Beschwerden auftauchen. Das Verfahren kann somit einem sehr großen praktischen Nutzen zugeführt werden.

Die Darstellungen gemäß den Fig. 8a sowie 8b zeigen Möglichkeiten der Anordnung eines Flächenabschnitts 24 zur Messung der Blickrichtung (Fixation) sowie eines auf die Netzhaut projizierten Testmusters 23 zur Messung der Akkommodation innerhalb eines Bereichs der Netzhaut 25 des menschlichen Auges.

Der Flächenabschnitt 24 beinhaltet bestimmte Charakteristika der Netzhaut 25, wie z.B. einzelne durch den Flächenabschnitt hindurch verlaufende Blutgefäße 27. Durch fototechnische Erfassung der Verschiebung dieser Charakteristika innerhalb des Flächenabschnitts 24 bedingt der Blickrichtung kann fototechnisch die Blickrichtung ermittelt werden. Der Flächenabschnitt 24 dient somit dazu, mittels einer digitalen Kamera erfasst und bildanalytisch über einen zeitlichen Verlauf ausgewertet zu werden.

Das auf die Netzhaut 25 projizierte Muster 23 liegt bei der Ausgestaltung nach Fig. 8a innerhalb des Flächenabschnitts 24, sodass mit einer einzigen digitalen Kamera bei entsprechender Umschaltung des Beleuchtungsmoduls 9 bzw. 14 (vgl. Fig. 2) eine einzige digitale Kamera zur Auswertung eines Auges verwendet werden kann.

Bei der Ausgestaltung nach Fig. 8b liegt das Testmuster 23 sowie der Flächenabschnitt 24 nebeneinander. Hierbei kann die Auswertung der Akkommodation sowie Blickrichtung unter Verwendung zweier digitaler Kameras gleichzeitig erfolgen.

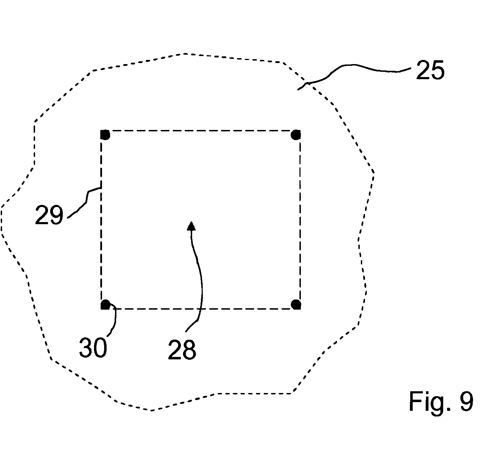

Die Fig. 9 zeigt eine Teilfläche der Netzhaut 25, in der sich die Makula 28 d.h. derjenige Bereich der Netzhaut 25 befindet, in dem die Anzahl der Sehzellen am häufigsten ist. Der Pfeil in Fig. 9 kennzeichnet das Zentrum der Makula 28. Gemäß der vorliegenden Erfindung kann zweckmäßigerweise ein Testbild 29 erzeugt werden, bei dem mehrere Objekte 30, z.B. vier Punkte als Ecken eines Quadrats, im Randbereich der Makula 28 positioniert werden. Ein Patient, der gegebenenfalls bei einem vorherigen Test ein im Zentrum der Makula 28 befindliches Objekt nicht scharf sehen konnte, kann mit dem Testbild 29 hinsichtlich seiner Sehfähigkeit im Randbereich der Makula 28 getestet werden. Ein Patient mit fehlendem foveolaren Sehen, d.h. mit einer Degeneration des Zentrums der Makula 28, aber intaktem parafoveolaren Sehen, d.h. mit noch keiner Degeneration im Randbereich der Makula 28, kann die vier Punkte erkennen und die Mitte des Quadrats anfixieren.

### BEZUGSZEICHENLISTE

- 1: Auge
- 2: Bilderzeugungsmodule
- 3: Abbildungsmodul
- 4: Akkommodationsmesseinrichtung
- 5: Blickrichtungsmesseinrichtung
- 6: Steuerungs- und Auswertemodul
- 7: Strahlengang
- 8: Strahlengang
- 9: Beleuchtungsmodul
- 10: Maske
- 11: Display
- 12: optisches Bauteil
- 13: Digitale Kamera
- 14: Beleuchtungsmodul
- 15: Computer
- 16: Okular
- 17: Kameraobjektiv
- 18: Fokussiereinheit
- 19: Ansteuereinheit
- 20: Spiegel
- 21: Strahlteiler
- 22: LED-Treiber
- 23: Testmuster
- 24: Flächenabschnitt auf Netzhaut
- 25: Netzhaut
- 26: dichroitischer Spiegel
- 27: Blutgefäß
- 28: Makula
- 29: Testbild
- 30: Objekt

## Patentansprüche

1. Vorrichtung zur Überprüfung der menschlichen Sehfähigkeit umfassend
ein Bilderzeugungsmodul (z. B. 2a) zur Erzeugung von Testbilder,
ein Abbildungsmodul (z. B.3a), welches dazu dient, das von dem Bilderzeugungsmodul (z. B. 2a) bereitgestellte Testbild als Stimulus auf der Netzhaut des Auges abzubilden, wobei das Abbildungsmodul (z. B. 3a) mindestens ein optisches Bauelement mit variabler Brennweite enthält, sodass das Testbild des Bilderzeugungsmoduls (z. B. 2a) für das Auge aus variablen Entfernungen wahrnehmbar ist,
eine Akkommodationsmesseinrichtung (z. B. 4a) zur Messung der Akkommodation des Auges,
eine Blickrichtungsmesseinrichtung (z. B. 5a) zur Messung der Blickrichtung des Auges,
ein Steuerungs- und Auswertemodul (6), welches die von den einzelnen Modulen stammenden Informationen und/oder Messwerte erfasst und/oder weiterverarbeitet und/oder den Betriebsablauf steuert,
**wobei** mittels des jeweiligen Testbilds eine Stimulation der Akkommodation des Auges durchführbar ist, mittels des jeweiligen Testbilds eine Stimulation der Blickrichtung durchführbar ist,
die Messung der Akkommodation des Auges sowie die Messung der Blickrichtung des Auges gleichzeitig oder alternierend durchführbar ist und
die Messwerte der Akkommodation des Auges sowie der Blickrichtung des Auges dem gemeinsamen Steuerungs- und Auswertemodul (6) zuführbar sind
**dadurch gekennzeichnet, dass** das Bilderzeugungsmodul (2a, 2b) ein Display (11) zur Erzeugung des Testbilds umfasst, welches vom Steuerungs- und Auswertemodul (6) ansteuerbar ist und das Testbild vom Display (11) in den Strahlengang (7a, 8a bzw. 7b, 8b) projizierbar ist und
es sich bei der Blickrichtungsmesseinrichtung (z. B. 5a) um eine ophthalmoskopische Blickrichtungsmesseinrichtung handelt, bei der die Bewegung der Netzhaut direkt erfasst wird.

2. Vorrichtung nach Anspruch 1 **dadurch gekennzeichnet, dass**
für jedes Auge (1a, 1b) getrennt mittels eines zugehörigen Bilderzeugungsmoduls (2a, 2b) ein Testbild erzeugbar ist, mittels eines zugehörigen Abbildungsmoduls (3a, 3b) eine Abbildung des Testbilds auf der Netzhaut des betreffenden Auges durchführbar ist, mittels einer zugehörigen Akkommodationsmesseinrichtung (4a, 4b) die Akkommodation des betreffenden Auges messbar ist und/oder mittels einer zugehörigen Blickrichtungsmesseinrichtung (5a, 5b) die Blickrichtung des betreffenden Auges messbar ist,
das Steuerungs- und Auswertemodul (6) die von den einzelnen Modulen stammenden Informationen und/oder Messwerte erfasst und/oder weiterverarbeitet und/oder den Betriebsablauf steuert,
mittels des jeweiligen Testbilds eine Stimulation der Akkommodation des jeweiligen Auges durchführbar ist, und/oder
mittels des jeweiligen Testbilds eine Stimulation der Konvergenz der Augen durchführbar ist,
die Messung der Akkommodation des jeweiligen Auges sowie die Messung der Blickrichtung des jeweiligen Auges gleichzeitig oder alternierend durchführbar ist und
die Messwerte der Akkommodation des jeweiligen Auges sowie der Blickrichtung des jeweiligen Auges dem gemeinsamen Steuerungs- und Auswertemodul (6) zuführbar sind.

3. Vorrichtung nach Anspruch 2, **dadurch gekennzeichnet, dass** je ein Bilderzeugungsmodul (2a bzw. 2b), je ein Abbildungsmodul (3a bzw. 3b), je eine Akkommodationsmesseinrichtung (4a bzw. 4b) und/oder je eine Blickrichtungsmesseinrichtung (5a bzw. 5b) so angeordnet sind, dass mit ihnen ein erster Strahlengang (7a, 8a) für das erste Auge (1a) und ein zweiter Strahlengang (7b, 8b) für das zweite Auge (1b) festgelegt sind.

4. Vorrichtung nach mindestens einem der vorhergehenden Ansprüche,
**dadurch gekennzeichnet, dass** eine fortlaufende Reihe von Messwerten erzeugbar ist.

5. Vorrichtung nach mindestens einem der vorhergehenden Ansprüche,
**dadurch gekennzeichnet, dass** für die Messung der Akkommodation und/oder der Blickrichtung eine Kamera, vorzugsweise eine digitale Kamera (13), vorzugsweise je eine Kamera für das erste und das zweite Auge (1a, 1b), vorgesehen ist.

6. Vorrichtung nach Anspruch 4, **dadurch gekennzeichnet, dass** eine Kamera gemeinsam für die Messung der Akkommodation und der Blickrichtung eines Auges vorgesehen ist und die Messwerte fortlaufend alternierend erzeugt werden.

7. Vorrichtung nach mindestens einem der vorhergehenden Ansprüche,
**dadurch gekennzeichnet, dass** für die Akkommodationsmesseinrichtung (4a bzw. 4b) ein erstes Beleuchtungsmodul (14) und für die Blickrichtungsmesseinrichtung (5a bzw. 5b) ein zweites Beleuchtungsmodul (9) vorgesehen sind.

8. Vorrichtung nach Anspruch 7, **dadurch gekennzeichnet, dass** im Beleuchtungsstrahlengang der Akkommodationsmesseinrichtung (4a bzw. 4b) eine Maske (10) zur Abbildung eines Testmusters auf dem Augenhintergrund vorgesehen ist.

9. Vorrichtung nach mindestens einem der vorhergehenden Ansprüche,
**dadurch gekennzeichnet, dass** in dem gemeinsamen Strahlengang (7a, 8a bzw. 7b, 8b) ein optisches Bauteil (12) zur Aperturanpassung vorgehen ist.

10. Vorrichtung nach mindestens einem der vorhergehenden Ansprüche,
**dadurch gekennzeichnet, dass** das in dem gemeinsamen Strahlengang (7a, 8a bzw. 7b, 8b) ein dichroitischer Spiegel (26) vorgehen ist.

11. Verfahren zur Überprüfung der menschlichen Sehfähigkeit umfassend folgende Verfahrensschritte:
Erzeugung eines isolierten Testbilds,
Abbilden des Testbilds auf der Netzhaut des ersten Auges,
Stimulation der Akkommodation durch Veränderung der Fokuslage des Testbilds innerhalb eines Bereichs vom Fernpunkt der Akkommodation bis zum Nahpunkt der Akkommodation sowie gegebenenfalls darüber hinaus
Messung der Akkommodation des Auges,
Messung der Blickrichtung (Fixation) des Auges,
wobei die Messung der Akkommodation des Auges sowie die Messung der Blickrichtung des Auges gleichzeitig oder fortlaufend alternierend durchgeführt wird und
die Messwerte der Akkommodation sowie der Blickrichtung des Auges einem Steuerungs- und Auswertemodul (6) zugeführt werden
**dadurch gekennzeichnet, dass**
das Bilderzeugungsmodul (2a, 2b) ein Display (11) zur Erzeugung des Testbilds umfasst, welches vom Steuerungs- und Auswertemodul (6) angesteuert wird,
das Testbild vom Display (11) in den Strahlengang (7a, 8a bzw. 7b, 8b) projiziert wird und
es sich bei der Blickrichtungsmesseinrichtung (z. B. 5a) um eine ophthalmoskopische Blickrichtungsmesseinrichtung handelt, bei der die Bewegung der Netzhaut direkt erfasst wird.

12. Verfahren nach Anspruch 11, **gekennzeichnet durch folgende Verfahrensschritte:**
getrennte Erzeugung eines isolierten ersten Testbilds sowie isolierten zweiten Testbilds für das erste Auge bzw. das zweite Auge,
Abbilden des ersten Testbilds auf der Netzhaut des ersten Auges sowie des zweiten Testbilds auf der Netzhaut des zweiten Auges,
für beide Augen getrennte Stimulation der Akkommodation **durch** Veränderung der Fokuslage des Testbilds innerhalb eines Bereichs vom Fernpunkt der Akkommodation bis zum Nahpunkt der Akkommodation sowie gegebenenfalls darüber hinaus vorzugsweise bei gleichzeitiger Variation des Konvergenzwinkels,
Messung der Akkommodation des ersten Auges sowie separate Messung der Akkommodation des zweiten Auges,
Messung der Blickrichtung (Fixation) des ersten Auges sowie separate Messung der Blickrichtung (Fixation) des zweiten Auges,
Berechnung des Konvergenzwinkels anhand der Messwerte der Blickrichtung beider Augen, wobei
die Messung der Akkommodation des jeweiligen Auges sowie die Messung der Blickrichtung des jeweiligen Auges gleichzeitig oder fortlaufend alternierend durchgeführt wird und
die Messwerte der Akkommodation des jeweiligen Auges sowie der Blickrichtung des jeweiligen Auges dem Steuerungs- und Auswertemodul (6) zugeführt werden.

13. Verfahren nach den Ansprüchen 11 oder 12, **dadurch gekennzeichnet, dass** zur Messung der Akkommodation ein Testmuster auf der Netzhaut des jeweiligen Auges abgebildet wird,
das Bild des Testmusters mittels einer Kamera, vorzugsweise einer digitalen Kamera, erfasst und bildanalytisch ausgewertet wird, und
die aktuelle Akkommodation des jeweiligen Auges unter Berücksichtigung der Einstellung der optischen Bauelemente mit variabler Lage bezüglich der Brennebene der Abbildungsmodule (3a bzw. 3b) ermittelt wird.

14. Verfahren nach den Ansprüchen 11 bis 13, **dadurch gekennzeichnet, dass** zur Messung der Blickrichtung (Fixation) ein Flächenabschnitt (24) auf der Netzhaut (25) des jeweiligen Auges ausgewählt wird und
der Flächenabschnitt (24) mittels einer Kamera erfasst und bildanalytisch ausgewertet wird.

15. Verfahren nach den Ansprüchen 12 bis 14, **dadurch gekennzeichnet, dass** der AC/A-Quotient und/oder CA/C-Quotient aus den aktuell gemessenen Werten der akkommodativen Konvergenz und der Akkommodation ermittelt wird.

16. Verfahren nach den Ansprüchen 11 bis 15, **dadurch gekennzeichnet, dass** das Ein- und Ausschalten der Stimuli für Akkommodation und/oder Konvergenz so gesteuert werden, dass vorzugsweise zeitaufgelöst die Änderungen der Akkommodation und/oder der Blickrichtungen der Augen erfasst werden, die bei plötzlichem Auftreten oder Wegfall eines oder mehrerer Stimuli oder aller Stimuli auftreten.

17. Verfahren nach den Ansprüchen 11 bis 16, **dadurch gekennzeichnet, dass** die Reaktionen der Augen auf die Stimuli als zeitliche Verläufe dargestellt werden.

## Claims

1. Device for testing human vision comprising
an image-generating module (e.g. 2a) for generating test images,
an imaging module (e.g. 3a), which is used to project the test image provided by the image-generating module (e.g. 2a) onto the retina of the eye as a stimulus, with the imaging module (e.g. 3a) containing at least one optical component having variable focal length, such that the test image of the image-generating module (e.g. 2a) is perceptible to the eye from variable distances,
an accommodation measuring apparatus (e.g. 4a) for measuring the accommodation of the eye,
a viewing direction measuring apparatus (e.g. 5a) for measuring the viewing direction of the eye,
a control and evaluation module (6), which records and/or further processes the information and/or measured values originating from the individual modules and/or controls the operation sequence,
**with** a stimulation of the accomodation of the eye by means of the respective test image being feasible, a stimulation of the viewing direction by means of the respective test image being feasible,
the measurement of the accommodation of the eye and the measurement of the viewing direction of the respective eye can be carried out simultaneously or alternately, and
the measured values of the accommodation of the eye and of the viewing direction of the eye can be fed to the common control and evaluation module (6),
**characterised in that** the image-generating module (2a, 2b) comprises a display (11) for generating the test image, which can be actuated by the control and evaluation module (6) and the test image can be projected by the display (11) into the beam path (7a, 8a or 7b, 8b) and
the viewing direction measuring apparatus (e.g. 5a) is an ophthalmologic viewing direction measuring apparatus, which directly records the movement of the retina.

2. Device according to claim 1, **characterised in that**
separately for each eye (1 a, 1 b) a test image can be generated by means of a corresponding image-generating module (2a, 2b), a projection of the test image onto the retina of the respective eye can be done by means of a corresponding imaging module (3a, 3b), the accommodation of the respective eye can be measured by means of a corresponding accommodation measuring apparatus (4a, 4b) and/or the viewing direction of the respective eye can be measured by a corresponding viewing direction measuring apparatus (5a, 5b),
the control and evaluation module (6) records and/or further processes the information and/or measured values originating from the individual modules and/or controls the operation sequence,
a stimulation of the accommodation of the respective eye can be done by means of the respective test image, and/or
a stimulation of the convergence of the eyes can be done by means of the respective test image,
the measurement of the accommodation of the respective eye and the measurement of the viewing direction of the respective eye can be done simultaneously or alternately, respectively, and
the measured values of the accommodation of the respective eye and the viewing direction of the respective eye can be fed to the common control and evaluation module (6).

3. Device according to claim 2, **characterised in that** one image-generating module (2a or 2b), one imaging module (3a or 3b), one accommodation measuring apparatus (4a or 4b) and/or one viewing direction measuring apparatus (5a or 5b) each are arranged so that they establish a first beam path (7a, 8a) for the first eye (1a) and a second beam path (7b, 8b) for the second eye (Ib).

4. Device according to at least one of the preceding claims, **characterised in that** a progressive series of measured values can be generated.

5. Device according to at least one of the preceding claims, **characterised in that** a camera is provided for the measurement of the accommodation and/or the viewing direction, preferably a digital camera (13), preferably one camera each for the first and the second eye (1a, 1b).

6. Device according to claim 4, **characterised in that** one camera is provided in common for the measurement of the accommodation and the viewing direction of one eye and the measured values are progressively generated alternately.

7. Device according to at least one of the preceding claims, **characterised in that** a first illumination module (14) is provided for the accommodation measuring apparatus (4a or 4b) and a second illumination module (9) for the viewing direction measuring apparatus (5a or 5b).

8. Device according to claim 7, **characterised in that** a mask (10) is provided in the illumination beam path of the accommodation measuring apparatus (4a or 4b) for projecting a test pattern onto the fundus of the eye.

9. Device according to at least one of the preceding claims, **characterised in that** an optical component (12) is provided in the common beam path (7a, 8a or 7b, 8b) for aperture adjustment.

10. Device according to at least one of the preceding claims, **characterised in that** a dichroic mirror (26) is provided in the common beam path (7a, 8a or 7b, 8b).

11. Process for testing human vision, comprising the following steps:
generating an isolated test image,
projecting the test image onto the retina of the first eye,
stimulation of the accommodation by changing the focus position of the test image within a region from the far point of the accommodation to the near point of the accommodation and possibly beyond
measuring the accommodation of the eye,
measuring the viewing direction (fixation) of the eye,
with the measuring of the accommodation of the eye and the measuring of the viewing direction of the eye being done simultaneously or progressively and alternately, respectively, and
the measured values of the accommodation and the viewing direction of the eye are fed to a control and evaluation module (6),
**characterised in that**
the image-generating module (2a, 2b) comprises a display (11) for generating the test image, which is actuated by the control and evaluation module (6),
the test image is projected by the display (11) into the beam path (7a, 8a or 7b) and the viewing direction measuring apparatus (e.g. 5a) is an ophthamoscopic viewing direction measuring apparatus which directly records the movement of the retina

12. Process according to claim 11, **charactersised by the following process steps:**
separate generating of an isolated first test image and isolated second test image for the first eye and the second eye, respectively,
projecting of the first test image onto the retina of the first eye and of the second test image onto the retina of the second eye,
for both eyes, separate stimulation of the accommodation by changing the focus position of the test image within a region from the far point of the accommodation to the near point of the accommodation and possibly beyond preferably under simultaneous variation of the convergence angle,
measuring of the accommodation of the first eye and separate measuring of the accommodation of the second eye,
measuring of the viewing direction (fixation) of the first eye and separate measuring of the viewing direction (fixation) of the second eye,
calculation of the convergence angle with the aid of the measured values of the viewing direction of the two eyes, with
the measuring of the accommodation of the respective eye and the measuring of the viewing direction of the respective eye being done simultaneously or progressively and
alternately, respectively, and
the measured values of the accommodation of the respective eye and the viewing direction of the respective eye being fed to the control and evaluation module (6).

13. Process according to claims 11 or 12, **characterised in that** a test pattern is projected onto the retina of the respective eye for measuring the accommodation,
the image of the test pattern is recorded by means of a camera, preferably a digital camera, and evaluated by image analysis, and
the current accommodation of the respective eye is determined with consideration of the setting of the optical components with variable position in regard to the focal plane of the imaging modules (3a or 3b).

14. Process according to claims 11 to 13, **characterised in that** a surface segment (24) onto the retina (25) of the respective eye is selected for measuring the viewing direction (fixation) and
the surface segment (24) is recorded by means of a camera and evaluated by image analysis.

15. Processes according to claims 12 to 14, **characterised in that** the AC/A quotient and/or CA/C quotient is determined from the currently measured values of the accommodative convergence and the accommodation.

16. Process according to claims 11 to 15, **characterised in that** the switching on and off of the stimuli for accommodation and/or convergence are controlled so that, preferably resolved over time, the changes of accommodation and/or the viewing directions of the eyes are recorded which occur upon a sudden occurrence or omission of one stimulus or more than one stimuli or all stimuli.

17. Process according to claims 11 to 16, **characterised in that** the reactions of the eyes to the stimuli are shown as temporal progressions.

## Revendications

1. Dispositif pour contrôler l'acuité visuelle humaine comprenant un module de formation d'images (p. ex. 2a) pour produire des images test,
un module d'imagerie (p. ex. 3a), qui sert à projeter l'image test produite par le module de formation d'images en tant que stimulus sur la rétine de l'oeil , étant entendu que le module d'imagerie (p. ex. 3a) comprend au moins un élément optique à focale variable pour que l'image test du module de formation d'images (p. ex. 2a) soit visible pour l'oeil à une distance variable,
un dispositif de mesure de l'accommodation (p. ex. 4a) pour mesurer l'accommodation de l'oeil,
un dispositif de mesure de la direction du regard (p. ex. 5a) pour mesurer la direction du regard de l'oeil,
un module de commande et d'analyse (6) qui recueille et/ou traite les informations et/ou les résultats de chaque module et/ou qui règle le bon fonctionnement, pendant qu'une stimulation de l'accommodation de l'oeil peut être réalisée à l'aide de l'image test concernée, une stimulation de la direction du regard peut être réalisée à l'aide de l'image test concernée,
la mesure de l'accommodation de l'oeil et celle de la direction du regard de l'oeil peuvent être réalisées en même temps ou alternativement et
les résultats de la mesure de l'accommodation de l'oeil et de la direction du regard de l'oeil peuvent être transférés au module commun de commande et d'analyse (6) caractérisé en cela que le module de formation d'images (2a, 2b) comprend un écran de visualisation (11) pour produire l'image test qui peut être activée par le module de commande et d'analyse (6) et l'image test de l'écran de visualisation (11) peut être projetée dans le champ visuel (7a, 8a ou 7b, 8b) et
le dispositif de mesure de la direction du regard (p. ex. 5a) est un dispositif de mesure de la direction du regard ophtalmoscope qui enregistre directement le mouvement de la rétine.

2. Dispositif selon la revendication 1 caractérisée en cela qu'une image test est productible pour chaque oeil (1a, 1 b) par un module de formation d'images (2a, 2b), une projection de l'image test sur la rétine de l'oeil concerné est réalisable par un module d'imagerie (3a, 3b), l'accommodation de l'oeil concerné est mesurable par un dispositif de mesure de l'accommodation (4a, 4b) et/ou la direction du regard de l'oeil concerné est mesurable par un dispositif de mesure du regard de l'oeil (5a, 5b),
le module de commande et d'analyse (6) qui recueille et/ou traite les informations et/ou les résultats de chaque module et/ou qui règle le bon fonctionnement,
par l'image test en question, l'accommodation de chaque oeil peut être stimulée, et/ou par l'image test en question, la convergence des yeux peut être stimulée,
la mesure de l'accommodation de chaque oeil et la mesure de la direction du regard de chaque oeil peuvent être réalisées en même temps ou alternativement et
les résultats de mesure de l'accommodation de chaque oeil et de la direction du regard de chaque oeil sont transférés au module commun de commande et d'analyse (6).

3. Dispositif selon la revendication 2 caractérisée en cela qu'un module de formation d'images (2a ou 2b), un module d'imagerie (3a ou 3b), un dispositif de mesure de l'accommodation (4a ou 4b) et/ou un dispositif de mesure de la direction du regard (5a ou 5b) sont chacun aménagés de telle façon que, grâce à eux, un premier champ visuel (7a, 8a) est défini pour le premier oeil (1a) et un deuxième champ visuel (7b, 8b) pour le deuxième oeil (1 b).

4. Dispositif selon au moins l'une des revendications précédentes caractérisée en cela qu'une série consécutive de résultats de mesure peut être produite.

5. Dispositif selon au moins l'une des revendications précédentes caractérisée en cela qu'une caméra, de préférence une caméra numérique (13), de préférence une caméra pour le premier et une pour le deuxième oeil (1a, 1 b), est prévue pour mesurer l'accommodation et/ou la direction du regard.

6. Dispositif selon la revendication 4 caractérisée en cela qu'une seule caméra est prévue pour mesurer l'accommodation et la direction du regard d'un oeil et les résultats de mesure sont continuellement produits en alternance.

7. Dispositif selon au moins l'une des revendications précédentes caractérisée en cela que le dispositif de mesure de l'accommodation (4a ou 4b) contient un premier module d'éclairage (14) et le dispositif de mesure de la direction du regard de l'oeil (5a ou 5b) contient un deuxième module d'éclairage (9).

8. Dispositif selon la revendication 7 caractérisée en cela que le faisceau lumineux du dispositif de mesure de l'accommodation (4a ou 4b) comprend un masque (10) pour projeter une image du motif de test sur le fond de l'oeil.

9. Dispositif selon au moins l'une des revendications précédentes caractérisée en cela que le champ visuel commun (7a, 8a ou 7b, 8b) comprend un élément optique (12) pour adapter l'ouverture.

10. Dispositif selon au moins l'une des revendications précédentes caractérisée en cela que le champ visuel commun (7a, 8a ou 7b, 8b) comprend un miroir dichroïque (26).

11. Méthode pour mesurer l'acuité visuelle humaine à l'aide des procédés suivantes :
production d'une image test isolée,
projection de l'image test sur la rétine du premier oeil,
stimulation de l'accommodation par le changement du plan focal de l'image test dans une zone du punctum remotum de l'accommodation dans une zone du punctum proximum de l'accommodation et éventuellement au-delà de cette zone,
mesure de l'accommodation de l'oeil,
mesure de la direction du regard (fixation) de l'oeil,
pendant laquelle la mesure de l'accommodation de l'oeil ainsi que la mesure de la direction du regard de l'oeil sont effectuées en même temps ou alternativement et
les résultats de mesure de l'accommodation et de la direction du regard de l'oeil sont transférés à un module de commande et d'analyse (6)
caractérisé en cela que le module de formation d'images (2a,2b) comprend un écran de visualisation (11) pour produire une image test régulée par le module de commande et d'analyse (6),
l'image test est projetée de l'écran de visualisation (11) dans le champ visuel (7a, 8a ou 7b, 8b) et
le dispositif de mesure de la direction du regard (p. ex. 5a) est un dispositif de mesure de la direction du regard ophtalmoscope qui mesure directement le mouvement de la rétine.

12. Méthode selon la revendication 11 **caractérisée par** des procédés suivantes :
production séparée d'une première image test isolée ainsi que d'une deuxième image test isolée pour le premier ou le deuxième oeil,
projection de la première image test sur la rétine du premier oeil ainsi que projection de la deuxième image test sur la rétine du deuxième oeil,
stimulation séparée de l'accommodation des yeux par un changement du plan focal de l'image test dans une zone du punctum remotum de l'accommodation au punctum proximum de l'accommodation ainsi qu'au-delà de cette zone éventuellement, de préférence en changeant l'angle de convergence en même temps,
mesure de l'accommodation du premier oeil ainsi que mesure séparée de l'accommodation du deuxième oeil,
mesure de la direction du regard (fixation) du premier oeil ainsi que mesure séparée de la direction du regard (fixation) du deuxième oeil,
calcul de l'angle de convergence à partir des résultats de mesure de la direction du regard des yeux
pendant lequel la mesure de l'accommodation de l'oeil ainsi que la mesure de la direction du regard de l'oeil sont effectuées en même temps ou alternativement et
les résultats de mesure de l'accommodation et de la direction du regard de chaque oeil sont transférés au module de commande et d'analyse (6).

13. Méthode selon les revendications 11 ou 12 caractérisée en cela qu'un motif de test est projeté sur la rétine de l'oeil concerné pour mesurer l'accommodation,
l'image du motif de test est saisie et analysée selon les images par une caméra, de préférence une caméra numérique, et
l'accommodation actuelle de l'oeil concerné est mesurée en tenant compte du réglage des éléments optiques à position variable au niveau du plan focal des modules d'imagerie (3a ou 3b).

14. Méthode selon les revendications 11 à 13 caractérisée en cela qu'une partie (24) de la rétine (25) de l'oeil concerné est choisie pour mesurer la direction du regard (fixation) et
la partie (24) est saisie par une caméra et analysée selon les images.

15. Méthode selon les revendications 12 à 14 caractérisée en cela que le quotient AC/A et/ou le quotient CA/C est déterminé à partir des résultats du moment des mesures de la convergence accommodative et de l'accommodation.

16. Méthode selon les revendications 11 à 15 caractérisée en cela que la projection ou l'effacement des stimuli pour l'accommodation et/ou la convergence sont réglées de manière à ce que les changements de l'accommodation et/ou de la direction du regard des yeux soient captés par les yeux, de préférence avec résolution temporelle,
qui apparaissent lors de l'apparition ou de la disparition soudaine d'un ou de plusieurs stimuli ou de tous les stimuli.

17. Méthode selon les revendications 11 à 16 caractérisée en cela que les réactions des yeux à des stimuli sont représentées comme des courbes temporelles.
